# EUROPEAN PATENT APPLICATION

(11) **EP 4 670 662 A1**
(43) Date of publication of application: **31.12.2025**
(21) Application number: 25176370.2
(22) Date of filing: 14.05.2025
(51) Int. Cl.: A61B 34/10, A61N 1/36

(54) **SIMULATION-BASED DETERMINATION OF AN INSERTION OF AN ELECTRODE LEAD WITHIN A COCHLEA**

(30) Priority: 27.06.2024 US 202418755912
(71) Applicant: Advanced Bionics LLC, Valencia, CA 91355 (US)
(72) Inventor: Geiger, Stephan, Ventura (US); Nauwelaers, Tim, Hannover (DE); Guillon, Pierre, Stäfa (CH); Avci, Ersin, Isernhagen (DE)
(74) Representative: Schwan Schorer & Partner mbB

(57) **Abstract**

An exemplary system comprises memory that stores instructions; and a processor communicatively coupled to the memory and configured to execute the instructions to perform a process. The process may comprise accessing a three-dimensional (3D) cochlea model representative of a cochlea of a patient within which an electrode lead included in a cochlear implant system is to be inserted during an electrode lead insertion procedure, accessing one or more 3D electrode lead models representative of one or more electrode leads configured to be inserted within the cochlea, simulating, based on the 3D cochlea model and the one or more 3D electrode lead models, one or more insertion configurations associated with insertion of the one or more electrode leads into the cochlea, and performing, based on the simulating, an operation associated with the electrode lead insertion procedure.

## Description

### BACKGROUND INFORMATION

Cochlear implant systems are used to provide, restore, and/or improve the sense of hearing to patients with severe or profound hearing loss. Conventional cochlear implant systems include various components configured to be implanted within a patient. For example, an electrode lead may be inserted into a cochlea of the patient and stimulation current may be applied by electrodes on the electrode lead as directed by a cochlear implant that is also surgically implanted within the patient. The insertion of an electrode lead into the cochlea of the patient is performed by way of a delicate surgical procedure referred to herein as an "electrode lead insertion procedure."

Electrode lead insertion procedures are generally difficult due to the variable structure of the human cochlea, which is in the shape of a spiral beginning at a base and ending at an apex. Improper insertion of a cochlear electrode lead can result in damage to the electrode lead, damage to the cochlear tissue, and/or improper electrode placement in the cochlea (e.g., translocation, tip foldover, etc.), which may cause an inferior hearing outcome for the patient.

### BRIEF DESCRIPTION OF THE DRAWINGS

The accompanying drawings illustrate various embodiments and are a part of the specification. The illustrated embodiments are merely examples and do not limit the scope of the disclosure. Throughout the drawings, identical or similar reference numbers designate identical or similar elements.
FIG. 1 shows an illustrative cochlear implant system.
FIG. 2 shows an illustrative configuration of the cochlear implant system of FIG. 1.
FIG. 3 shows an illustrative configuration including an electrode lead simulation system.
FIG. 4 shows an illustrative method for performing a simulation based on a cochlea model and an electrode lead model.
FIG. 5 shows an illustrative flow diagram depicting operations that may be performed for generating a cochlea model.
FIG. 6 shows an illustrative implementation of a cochlea model.
FIG. 7 shows an illustrative flow diagram depicting operations that may be performed to simulate insertion of an electrode lead model within a cochlea model.
FIG. 8 shows an illustrative implementation of an electrode lead model being inserted within a cochlea model.
FIG. 9 shows an illustrative method for determining a type of electrode lead to be inserted within a cochlea.
FIG. 10 shows an illustrative flow diagram depicting operations that may be performed for determining a type of electrode lead to be inserted within a cochlea.
FIG. 11 shows an illustrative method for determining an insertion depth of an electrode lead to be inserted within a cochlea.
FIGS. 12A-12B show illustrative implementations for determining a first insertion depth of an electrode lead to be inserted within a cochlea.
FIG. 13 shows an illustrative implementation of an electrode lead positioned at a first insertion depth.
FIGS. 14A-14B show illustrative implementations for determining a second insertion depth of an electrode lead to be inserted within a cochlea.
FIG. 15 shows an exemplary computing device.

### DETAILED DESCRIPTION

Systems and methods for simulating the insertion of an electrode lead within a cochlea prior to an electrode lead insertion procedure are described herein. An exemplary system comprises memory that stores instructions and a processor communicatively coupled to the memory and configured to execute the instructions to perform a process. The process may comprise accessing a three-dimensional (3D) cochlea model representative of a cochlea of a patient within which an electrode lead included in a cochlear implant system is to be inserted during an electrode lead insertion procedure, accessing one or more 3D electrode lead models representative of one or more electrode leads configured to be inserted within the cochlea, the one or more 3D electrode lead models including one or more properties of the one or more electrode leads, simulating, based on the 3D cochlea model and the one or more 3D electrode lead models, one or more insertion configurations associated with insertion of the one or more electrode leads into the cochlea, and performing, based on the simulating, an operation associated with the electrode lead insertion procedure (e.g., in a patient bilaterally or unilaterally). The operation may include determining an insertion configuration of the electrode lead for the electrode lead insertion procedure, providing insertion configuration information for the electrode lead insertion procedure, and/or selecting a type of electrode lead for the electrode lead insertion procedure.

The systems and methods described herein may provide various benefits to cochlear implant patients, as well as others involved with managing cochlear implant systems. For example, the systems and methods such as those described herein may facilitate determining one or more insertion parameters (e.g., a type of the electrode lead, a rotation of the electrode lead, an insertion depth of the electrode lead, an insertion angle of the electrode lead, an insertion speed of the electrode lead, etc.) for inserting the electrode lead during the electrode lead insertion procedure. As a result, systems and methods such as those described herein may reduce trauma in the inner structure of the cochlea (e.g., damage to the tissue of the cochlea, the basilar membrane, spiral ganglion cells, etc.) and/or reduce mispositioning of the electrode lead (e.g., the electrode lead is not adequately within the cochlea, has translocated the basilar membrane, tip fold over, etc.) during the electrode lead insertion procedure, which may allow for an increased hearing outcome for the patient. In addition, by simulating the insertion of the electrode lead in the cochlea, it may be possible to optimize insertion parameters and/or electrode leads for a particular patient, which may improve an efficiency and/or quality of the electrode lead insertion procedure.

Various embodiments will now be described in more detail with reference to the figures. The disclosed systems and methods may provide one or more of the benefits mentioned above and/or various additional and/or alternative benefits that will be made apparent herein.

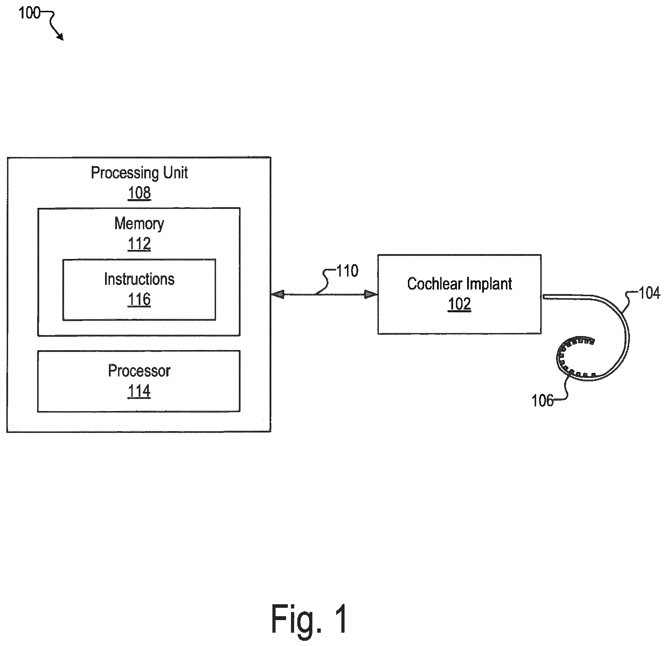

FIG. 1 illustrates an exemplary cochlear implant system 100 configured to be used by a patient. As shown, cochlear implant system 100 includes a cochlear implant 102, an electrode lead 104 physically coupled to cochlear implant 102 and having an array of electrodes 106, and a processing unit 108 configured to be communicatively coupled to cochlear implant 102 by way of a communication link 110.

The cochlear implant system 100 shown in FIG. 1 is unilateral (i.e., associated with only one ear of the patient). Alternatively, a bilateral configuration of cochlear implant system 100 may include separate cochlear implants and electrode leads for each ear of the patient. In the bilateral configuration, processing unit 108 may be implemented by a single processing unit configured to interface with both cochlear implants or by two separate processing units each configured to interface with a different one of the cochlear implants.

Cochlear implant 102 may be implemented by any suitable type of implantable stimulator. For example, cochlear implant 102 may be implemented by an implantable cochlear stimulator. Additionally or alternatively, cochlear implant 102 may be implemented by a brainstem implant and/or any other type of device that may be implanted within the patient and configured to apply electrical stimulation to one or more stimulation sites located along an auditory pathway of the patient.

In some examples, cochlear implant 102 may be configured to generate electrical stimulation representative of an audio signal processed by processing unit 108 in accordance with one or more stimulation parameters transmitted to cochlear implant 102 by processing unit 108. Cochlear implant 102 may be further configured to apply the electrical stimulation to one or more stimulation sites (e.g., one or more intracochlear locations) within the patient by way of one or more electrodes 106 on electrode lead 104. In some examples, cochlear implant 102 may include a plurality of independent current sources each associated with a channel defined by one or more of electrodes 106. In this manner, different stimulation current levels may be applied to multiple stimulation sites simultaneously by way of multiple electrodes 106.

Cochlear implant 102 may additionally or alternatively be configured to generate, store, and/or transmit data. For example, cochlear implant may use one or more electrodes 106 to record one or more signals (e.g., one or more voltages, impedances, evoked responses within the patient, and/or other measurements) and transmit, by way of communication link 110, data representative of the one or more signals to processing unit 108. In some examples, this data is referred to as back telemetry data.

Electrode lead 104 may be implemented in any suitable manner. For example, a distal portion of electrode lead 104 may be pre-curved such that electrode lead 104 conforms with the helical shape of the cochlea after being implanted. Electrode lead 104 may alternatively be naturally straight or of any other suitable configuration.

In some examples, electrode lead 104 includes a plurality of wires (e.g., within an outer sheath) that conductively couple electrodes 106 to one or more current sources within cochlear implant 102. For example, if there are n electrodes 106 on electrode lead 104 and n current sources within cochlear implant 102, there may be n separate wires within electrode lead 104 that are configured to conductively connect each electrode 106 to a different one of the n current sources. Exemplary values for n are 8, 12, 16, or any other suitable number.

Electrodes 106 are located on at least a distal portion of electrode lead 104. In this configuration, after the distal portion of electrode lead 104 is inserted into the cochlea, electrical stimulation may be applied by way of one or more of electrodes 106 to one or more intracochlear locations. One or more other electrodes (e.g., including a ground electrode, not explicitly shown) may also be disposed on other parts of electrode lead 104 (e.g., on a proximal portion of electrode lead 104) to, for example, provide a current return path for stimulation current applied by electrodes 106 and to remain external to the cochlea after the distal portion of electrode lead 104 is inserted into the cochlea. Additionally or alternatively, a housing of cochlear implant 102 may serve as a ground electrode for stimulation current applied by electrodes 106. In certain examples, electrode lead 104 may alternatively be referred to as an electrode array.

Processing unit 108 may be configured to interface with (e.g., control and/or receive data from) cochlear implant 102. For example, processing unit 108 may transmit commands (e.g., stimulation parameters and/or other types of operating parameters in the form of data words included in a forward telemetry sequence) to cochlear implant 102 by way of communication link 110. Processing unit 108 may additionally or alternatively provide operating power to cochlear implant 102 by transmitting one or more power signals to cochlear implant 102 by way of communication link 110. Processing unit 108 may additionally or alternatively receive data from cochlear implant 102 by way of communication link 110. Communication link 110 may be implemented by any suitable number of wired and/or wireless bidirectional and/or unidirectional links.

As shown, processing unit 108 includes a memory 112 and a processor 114 configured to be selectively and communicatively coupled to one another. In some examples, memory 112 and processor 114 may be distributed between multiple devices and/or multiple locations as may serve a particular implementation.

Memory 112 may be implemented by any suitable non-transitory computer-readable medium and/or non-transitory processor-readable medium, such as any combination of non-volatile storage media and/or volatile storage media. Exemplary non-volatile storage media include, but are not limited to, read-only memory, flash memory, a solid-state drive, a magnetic storage device (e.g., a hard drive), ferroelectric random-access memory ("RAM"), and an optical disc. Exemplary volatile storage media include, but are not limited to, RAM (e.g., dynamic RAM).

Memory 112 may maintain (e.g., store) executable data used by processor 114 to perform one or more of the operations described herein. For example, memory 112 may store instructions 116 that may be executed by processor 114 to perform any of the operations described herein. Instructions 116 may be implemented by any suitable application, program (e.g., sound processing program), software, code, and/or other executable data instance. Memory 112 may also maintain any data received, generated, managed, used, and/or transmitted by processor 114.

Processor 114 may be configured to perform (e.g., execute instructions 116 stored in memory 112 to perform) various operations with respect to cochlear implant 102.

To illustrate, processor 114 may be configured to control an operation of cochlear implant 102. For example, processor 114 may receive an audio signal (e.g., by way of a microphone communicatively coupled to processing unit 108, a wireless interface (e.g., a Bluetooth interface), and/or a wired interface (e.g., an auxiliary input port)). Processor 114 may process the audio signal in accordance with a sound processing program (e.g., a sound processing program stored in memory 112) to generate appropriate stimulation parameters. Processor 114 may then transmit the stimulation parameters to cochlear implant 102 to direct cochlear implant 102 to apply electrical stimulation representative of the audio signal to the patient.

In some implementations, processor 114 may also be configured to apply acoustic stimulation to the patient. For example, a receiver (also referred to as a loudspeaker) may be optionally coupled to processing unit 108. In this configuration, processor 114 may deliver acoustic stimulation to the patient by way of the receiver. The acoustic stimulation may be representative of an audio signal (e.g., an amplified version of the audio signal), configured to elicit an evoked response within the patient, and/or otherwise configured. In configurations in which processor 114 is configured to both deliver acoustic stimulation to the patient and direct cochlear implant 102 to apply electrical stimulation to the patient, cochlear implant system 100 may be referred to as a bimodal hearing system and/or any other suitable term.

Processor 114 may be additionally or alternatively configured to receive and process data generated by cochlear implant 102. For example, processor 114 may receive data representative of a signal recorded by cochlear implant 102 using one or more electrodes 106 and, based on the data, adjust one or more operating parameters of processing unit 108. Additionally or alternatively, processor 114 may use the data to perform one or more diagnostic operations with respect to cochlear implant 102 and/or the patient.

Other operations may be performed by processor 114 as may serve a particular implementation. In the description provided herein, any references to operations performed by processing unit 108 and/or any implementation thereof may be understood to be performed by processor 114 based on instructions 116 stored in memory 112.

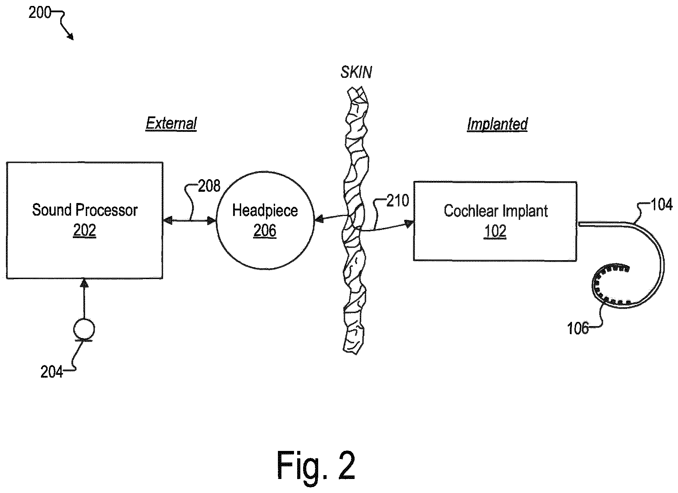

Processing unit 108 may be implemented by one or more devices configured to interface with cochlear implant 102. To illustrate, FIG. 2 shows an exemplary configuration 200 of cochlear implant system 100 in which processing unit 108 is implemented by a sound processor 202 configured to be located external to the patient. In configuration 200, sound processor 202 is communicatively coupled to a microphone 204 and to a headpiece 206 that are both configured to be located external to the patient.

Sound processor 202 may be implemented by any suitable device that may be worn or carried by the patient. For example, sound processor 202 may be implemented by a behind-the-ear ("BTE") unit configured to be worn behind and/or on top of an ear of the patient. Additionally or alternatively, sound processor 202 may be implemented by an off-the-ear unit (also referred to as a body worn device) configured to be worn or carried by the patient away from the ear. Additionally or alternatively, at least a portion of sound processor 202 is implemented by circuitry within headpiece 206.

Microphone 204 is configured to detect one or more audio signals (e.g., that include speech and/or any other type of sound) in an environment of the patient. Microphone 204 may be implemented in any suitable manner. For example, microphone 204 may be implemented by a microphone that is configured to be placed within the concha of the ear near the entrance to the ear canal, such as a T-MIC^{™} microphone from Advanced Bionics. Such a microphone may be held within the concha of the ear near the entrance of the ear canal during normal operation by a boom or stalk that is attached to an ear hook configured to be selectively attached to sound processor 202. Additionally or alternatively, microphone 204 may be implemented by one or more microphones in or on headpiece 206, one or more microphones in or on a housing of sound processor 202, one or more beam-forming microphones, and/or any other suitable microphone as may serve a particular implementation.

Headpiece 206 may be selectively and communicatively coupled to sound processor 202 by way of a communication link 208 (e.g., a cable or any other suitable wired or wireless communication link), which may be implemented in any suitable manner. Headpiece 206 may include an external antenna (e.g., a coil and/or one or more wireless communication components) configured to facilitate selective wireless coupling of sound processor 202 to cochlear implant 102. Headpiece 206 may additionally or alternatively be used to selectively and wirelessly couple any other external device to cochlear implant 102. To this end, headpiece 206 may be configured to be affixed to the patient's head and positioned such that the external antenna housed within headpiece 206 is communicatively coupled to a corresponding implantable antenna (which may also be implemented by a coil and/or one or more wireless communication components) included within or otherwise connected to cochlear implant 102. In this manner, stimulation parameters and/or power signals may be wirelessly and transcutaneously transmitted between sound processor 202 and cochlear implant 102 by way of a wireless communication link 210.

In configuration 200, sound processor 202 may receive an audio signal detected by microphone 204 by receiving a signal (e.g., an electrical signal) representative of the audio signal from microphone 204. Sound processor 202 may additionally or alternatively receive the audio signal by way of any other suitable interface as described herein. Sound processor 202 may process the audio signal in any of the ways described herein and transmit, by way of headpiece 206, stimulation parameters to cochlear implant 102 to direct cochlear implant 102 to apply electrical stimulation representative of the audio signal to the patient.

In an alternative configuration, sound processor 202 may be implanted within the patient instead of being located external to the patient. In this alternative configuration, which may be referred to as a fully implantable configuration of cochlear implant system 100, sound processor 202 and cochlear implant 102 may be combined into a single device or implemented as separate devices configured to communicate one with another by way of a wired and/or wireless communication link. In a fully implantable implementation of cochlear implant system 100, headpiece 206 may not be included and microphone 204 may be implemented by one or more microphones implanted within the patient, located within an ear canal of the patient, and/or external to the patient.

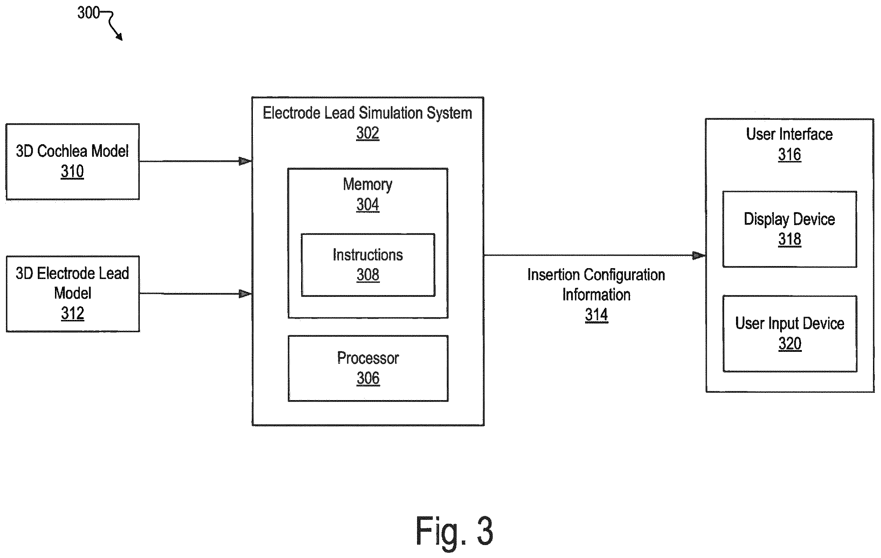

FIG. 3 shows an illustrative configuration 300 including an electrode lead simulation system 302 ("system 302"). As shown, system 302 includes, without limitation, a memory 304 and a processor 306 selectively and communicatively coupled to one another. Memory 304 and processor 306 may each include or be implemented by hardware and/or software components (e.g., processors, memories, communication interfaces, instructions stored in memory for execution by the processors, etc.). In some examples, memory 304 and/or processor 306 may be implemented by any suitable computing device. In other examples, memory 304 and/or processor 306 may be distributed between multiple devices and/or multiple locations as may serve a particular implementation. Illustrative implementations of system 302 are described herein.

Memory 304 may maintain (e.g., store) executable data used by processor 306 to perform any of the operations described herein. For example, memory 304 may store instructions 308 that may be executed by processor 306 to perform any of the operations described herein. Instructions 308 may be implemented by any suitable application, software, code, and/or other executable data instance.

Memory 304 may also maintain any data received, generated, managed, used, and/or transmitted by processor 306. Memory 304 may store any other suitable data as may serve a particular implementation. For example, memory 304 may store data associated with pre-operative scan images, post-operative scan images, ASM data, volumes of interest data, electrode lead graph data, graph matching parameters, cochlea landmark data, cochlea model database information, 3D cochlea model data, cochlear implant information (e.g., electrode lead dimensions information), graphical user interface content, and/or any other suitable data.

Processor 306 may be configured to perform (e.g., execute instructions 308 stored in memory 304 to perform) various processing operations associated with performing an operation associated with an electrode lead insertion procedure. For example, processor 306 may perform one or more operations described herein to simulate one or more insertion configurations associated with insertion of one or more electrode leads within a cochlea. These and other operations that may be performed by processor 306 are described herein.

System 302 may be implemented in any suitable manner. For example, system 302 may be implemented by any suitable computing device (e.g., a desktop computer, a laptop computer, a cloud computing device, etc.). In some examples, system 302 may be implemented by a computing device that represents an electrode lead insertion device configured to be selectively used (e.g., by a clinician) to insert electrode lead 104 of cochlear implant 102 within a cochlea of the patient. Additionally or alternatively, system 302 may be implemented by a computing device that represents a fitting device configured to be selectively used (e.g., by a clinician) to fit sound processor 202 and/or cochlear implant 102 to the patient (e.g., the computing device may be configured to execute a fitting program configured to set one or more operating parameters of sound processor 202 and/or cochlear implant 102 to values that are optimized for the patient). In these examples, the computing device may be considered to be separate from cochlear implant system 100 such that the computing device may be selectively coupled to cochlear implant system 100 when it is desired. Alternatively, system 302 may be included within cochlear implant system 100 (e.g., processing unit 108).

System 302 is configured to access one or more 3D cochlea models 310 each representative of a cochlea of a patient within which electrode lead 104 included in cochlear implant system 100 is to be inserted during an electrode lead insertion procedure. For example, each 3D cochlea model 310 may include an active shape-based 3D cochlea model. As used herein, an "active shape model" (ASM) is a statistical shape model (SSM) of a shape (e.g., a cochlea) that deforms to fit an example of that shape. An SSM is a geometric model that describes a collection of semantically similar shapes in a compact way and may be composed of an average shape as well as the main modes of shape variations. An ASM may be generated in any suitable manner. For example, an ASM may be generated based on high resolution scan images acquired from temporal bones of donors. In such examples, donors may be used because the high radiation doses associated with high resolution scan images may be too dangerous for a living patient.

The ASM-based 3D cochlea model may represent an ASM that has been deformed to fit one or more determined or estimated surfaces of the cochlea such as a surface of the cochlea wall and/or a surface of the basilar membrane, resulting in an estimation of the anatomical structure of the cochlea. In certain examples, the ASM-based 3D cochlea model may be specific to a particular patient of a cochlear implant.

In certain examples, the ASM-based 3D cochlea model may be previously generated based on pre-operative scan images (e.g., low resolution pre-operative CT scan images). In such examples, system 302 may access the ASM-based 3D cochlea model in any suitable manner from any suitable source. In certain alternative examples, system 302 may generate the ASM-based 3D cochlea model in any suitable manner based on pre-operative scan images of a patient and/or post-operative scan images of the patient.

System 302 is further configured to access one or more 3D electrode lead models 312 representative of one or more electrode leads 104 configured to be inserted within the cochlea (e.g., during an electrode lead insertion procedure). The one or more 3D electrode lead models 312 may include one or more properties of the one or more electrode leads 104. For example, the one or more properties may include, but is not limited to, one or more of a type of the one or more electrodes leads 104, a shape of the one or more electrode leads 104, a stiffness associated with the one or more electrode leads 104, a material associated with the one or more electrode leads 104, a size associated with the one or more electrode leads 104, one or more elastic moduli associated with the one or more electrode leads 104, a shear stress associated with the one or more electrode leads 104, or a friction associated with the one or more electrode leads 104.

To illustrate, the one or more electrode leads 104 may include one or more types of electrode leads 104, such as perimodiolar, lateral wall, and/or mid-scala electrode leads. Perimodiolar electrode leads may be configured to hug a modiolus wall of the cochlea. Lateral wall electrode leads may be configured to be placed along a lateral wall of a scala tympani (ST) of the cochlea. Mid-scala electrode leads may be configured to be implanted in the middle of the scala tympani. These types of electrode leads may include a straight or pre-curved configuration. In some implementations, pre-curved electrode leads are manufactured in an already-curled shape and are straightened before insertion using either a stylet that is inserted into a lumen of the pre-curved cochlear electrode lead or by using a straight rigid sheath provided around the pre-curved cochlear electrode lead. As such, the one or more 3D electrode lead models 312 may further include the stylet or sheath used for insertion of the one or more electrode leads 104.

In certain examples, the one or more 3D electrode lead models 312 may be previously generated. In such examples, system 302 may access the one or more 3D electrode lead models 312 in any suitable manner from any suitable source. In certain alternative examples, system 302 may generate the one or more 3D electrode lead models 312 in any suitable manner (e.g., based on images and/or properties of the electrode leads) as will be discussed in more detail below.

System 302 is further configured to simulate insertion of one or more electrode leads 104 into the cochlea using one or more 3D cochlea models 310 and one or more 3D electrode lead models 312. For example, the simulation may involve inserting one or more electrode leads 104 included in the one or more 3D electrode lead models 312 through an entry point (e.g., within a round window or cochleostomy of 3D cochlea model 310, or another suitable location) and into a scala tympani of 3D cochlea model 310. Each 3D electrode lead model 312 may generally follow a trajectory of a lateral wall of the scala tympani. In instances where the 3D electrode lead model 312 includes a pre-curved electrode lead, the simulation may include inserting the 3D electrode lead model 312 with a stylet or sheath that is gradually withdrawn during the insertion such that the pre-curved electrode lead returns to its curled shape and conforms with the helical shape of the cochlea away from the lateral wall and toward a modiolus of the cochlea.

System 302 may be configured to simulate multiple insertion configurations associated with insertion of one or more electrode leads 104 into the cochlea according to variety of insertion parameters. The insertion parameters may include, but are not limited to, one or more of a type of the one or more electrode leads 104, a rotation of the one or more electrode leads 104, an insertion depth of the one or more electrode leads 104, an insertion angle of the one or more electrode leads 104, or an insertion speed of the one or more electrode leads 104. For example, the rotation of the one or more electrode leads 104 may include a rotation of the one or more electrode leads 104 during insertion of the one or more electrode leads 104 within the cochlea in one or more directions (e.g., clockwise and/or counterclockwise) relative to the cochlea. The insertion depth of the one or more electrode leads 104 may include a depth (e.g., a distance, a degree, etc.) of the one or more electrode leads 104 inserted within the cochlea at one or more points (e.g., a point at which the one or more electrode leads 104 are released from a stylet or sheath, are positioned in a final placement, contact a wall of the cochlea, etc.) during the insertion of the one or more electrode leads 104 within the cochlea. The insertion angle of the one or more electrode leads 104 may include a trajectory of the one or more electrode leads 104 such as relative to a round window of the cochlea. The insertion speed of the one or more electrodes leads 104 may include a speed at which the one or more electrode leads 104 are inserted within the cochlea (e.g., in a translational direction toward the cochlea and/or in a rotational direction relative to the cochlea).

As an illustrative example, system 302 may simulate a first insertion configuration that includes inserting a first 3D electrode lead model 312 into 3D cochlea model 310 with a first set of one or more insertion parameters (e.g., a first rotation, a first insertion depth, and a first insertion angle). System 302 may further simulate additional insertion configurations that include inserting the first 3D electrode lead model 312 into 3D cochlea model 310 according to one or more other insertion parameters that are different than the first set of one or more insertion parameters. System 302 may additionally or alternatively simulate additional insertion configurations that include inserting one or more other 3D electrode lead models 312 into 3D cochlea model 310 according to the first set of one or more insertion parameters and/or one or more other insertion parameters.

Based on the simulations, system 302 may be configured to determine insertion configuration information 314 associated with the electrical lead insertion procedure. For example, the determining insertion configuration information 314 may include determining an insertion configuration to be used during the electrical lead insertion procedure having one or more insertion parameters configured to reduce trauma (e.g., damage) to the cochlea and/or accurately position a select electrode lead 104 within the cochlea. System 302 may be configured to provide insertion configuration information 314 for display to a user. For example, system 302 may direct a user interface 316 to display insertion configuration information 314 with a display device 318 of user interface 316. Display device 318 may be implemented by a monitor or other suitable device configured to display information to the user. In some examples, user interface 316 includes a user input device 320 that may be implemented by any suitable device or devices (e.g., a button, joystick, touchscreen, keyboard, handle, microphone, etc.) configured to receive a user input, for example, to interact with the display presented by display device 318 and/or designate one or more insertion parameters of the electrode lead insertion procedure.

In certain examples, the methods described herein may be performed automatically by system 302. As used herein, the expression "automatically" means that an operation or series of operations are performed without requiring further input from a user. For example, system 302 may automatically perform any of the operations described herein based on one or more 3D cochlea models 310 and/or 3D electrode lead models 312 without requiring further input from a user.

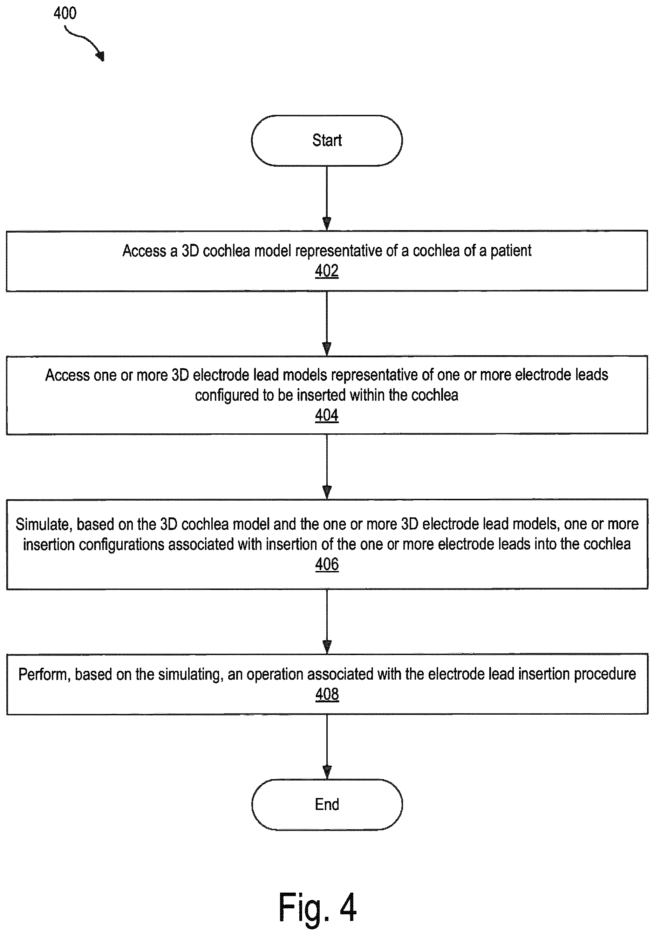

FIG. 4 illustrates an illustrative method 400 for simulating an electrode lead insertion procedure. While FIG. 4 illustrates exemplary operations according to one embodiment, other embodiments may omit, add to, reorder, and/or modify any of the operations shown in FIG. 4. Moreover, each of the operations depicted in Fig. 4 may be performed in any of the ways described herein. One or more of the operations depicted in Fig. 4 may be performed by cochlear implant system 100 and/or system 302.

Method 400 may include, at operation 402, accessing a 3D cochlea model (e.g., 3D cochlea model 310) representative of a cochlea of a patient within which an electrode lead (e.g., electrode lead 104) included in a cochlear implant system (e.g., cochlear implant system 100) is to be inserted during an electrode lead insertion procedure. The 3D cochlea model may include at least a portion of the cochlea, such as from a base (e.g., a basal region) of the cochlea to an apex (e.g., an apical region) of the cochlea. The 3D cochlea model may further define an entry point, such as a round window or cochleostomy of the cochlea. The accessing the 3D cochlea model may include accessing a previously generated 3D cochlea model and/or generating the 3D cochlea model.

Method 400 may further include, at operation 404, accessing one or more 3D electrode lead models (e.g., electrode lead model 312) representative of one or more electrode leads configured to be inserted within the cochlea. Each 3D electrode lead model may include at least a portion of each electrode lead such as at least a portion of the electrode lead comprising the plurality of electrodes. Each 3D electrode lead model further includes one or more properties of the electrode lead included in the 3D electrode lead model, such as a type of the electrodes leads, a shape of the electrode lead, a stiffness associated with the electrode lead, one or more materials associated with the electrode leads, a size associated with the electrode lead, one or more elastic moduli associated with the electrode lead, a shear stress associated with the electrode lead, and/or a friction associated with the electrode lead. The accessing the one or more 3D electrode lead models may include accessing a previously generated 3D electrode lead model and/or generating the 3D electrode lead model.

Method 400 may further include, at operation 406, simulating, based on the 3D cochlea model and the one or more 3D electrode lead models, one or more insertion configurations associated with insertion of the one or more electrode leads into the cochlea. The simulating the one or more insertion configurations may include performing one or more simulations such that each simulation is associated an insertion configuration having a set of one or more insertion parameters (e.g., a type, a shape, a size, a rotation, an insertion depth, an insertion angle, an insertion speed, etc.) of the electrode lead included in the simulation. In some examples, the simulating the one or more insertion configurations may include determining one or more insertion parameters for the simulation such as based on the 3D cochlea model, the one or more 3D electrode lead models and/or a user input (e.g., designated by way of user input device 320). Moreover, the one or more insertion parameters included in the simulation may be varied or adjusted during the simulation and/or between simulations (e.g., prior to each simulation).

As an illustrative example, the user input may designate one or more insertion parameters, such as a type, a size, and/or a shape of the electrode lead to be inserted during the electrode lead insertion procedure. Accordingly, one or more 3D electrode lead models associated with the type, size, and/or shape of the designated electrode lead may be accessed, and the simulating may include inserting each of the accessed one or more 3D electrode lead models into the 3D cochlea model. The simulating may further include selecting and/or adjusting one or more additional insertion parameters, such as a rotation, a speed, an insertion angle, and/or an insertion depth of the one or more 3D electrode lead models.

In some examples, the simulating the one or more insertion configurations may further include determining trauma to the cochlea associated with each insertion configuration. The determining the trauma may include, but is not limited to, determining one or more of damage to the inner structure of the cochlea, frictional stress to an outer wall of the cochlea, or deflection of the basilar membrane of the cochlea based on inserting the 3D electrode lead model within the 3D cochlea model. In some instances, the trauma may be determined relative to an insertion depth of the 3D electrode lead model within the 3D cochlea model (e.g., the trauma may be determined over multiple insertion depths as the 3D electrode lead model is inserted within the 3D cochlea model).

Method 400 may further include, at operation 408, performing, based on the simulating, an operation associated with the electrode lead insertion procedure. The operation may include selecting an insertion configuration of the one or more insertion configurations for the electrode lead insertion procedure. For example, the electrode lead associated with the 3D electrode lead model having the selected insertion configuration may be used during electrode lead insertion procedure. Additionally, the electrode lead may be inserted during the electrode lead insertion procedure according to the one or more insertion parameters included in the selected insertion configuration. To illustrate, the electrode lead may be inserted within the cochlea during the electrode lead insertion procedure at a rotation, a speed, an insertion angle, and/or an insertion depth associated with the selected insertion configuration.

In some examples, the insertion configuration may be selected based on one or more insertion factors associated with the insertion configuration during the simulation. The insertion factors may include, but is not limited to, one or more of an insertion angle of the electrode lead, an anatomy of the cochlea (e.g., a height of the scala tympani, a cochlear duct length, a cochlear radius, etc.), a proximity of a facial nerve to a position of the electrode lead, or trauma to the cochlea. To illustrate, the selecting the insertion configuration may be based on reducing trauma to the cochlea. As such, the insertion configuration associated with a minimum amount of trauma (e.g., an amount of trauma to the 3D cochlea model that is less than an amount of trauma to the 3D cochlea model associated with another insertion configuration) during the simulating may be selected for the electrode lead insertion procedure. Additionally or alternatively, the selecting the insertion configuration may be based on obtaining an adequate position of the 3D electrode lead model within the 3D cochlea model during the simulation (e.g., to avoid tip folder, translocation, damage to the electrode lead, etc.).

The operation may additionally or alternatively include prioritizing the one or more insertion configurations, such as based on the one or more insertion factors. As an illustrative example, the prioritizing may be based on reducing trauma to the cochlea such that a first insertion configuration associated with less trauma to the cochlea than a second insertion configuration is prioritized above the second insertion configuration. In certain scenarios, the prioritizing the one or more insertion configurations may include weighting the one or more factors associated with the one or more insertion configurations. For example, the reducing trauma to the cochlea may be weighted more than the position of the electrode lead such that an insertion configuration associated with a lower amount of trauma to the cochlea may be selected over another insertion configuration associated with an improved position of the electrode lead.

The operation may additionally or alternatively include providing insertion configuration information for the electrode lead insertion procedure. The insertion configuration information may include information associated with an insertion configuration for the electrode lead insertion procedure, such as the one or more insertion parameters (e.g., a type of the electrode lead to be inserted, a rotation of the electrode lead to be inserted, an insertion depth of the electrode lead to be inserted, an insertion angle of the electrode lead to be inserted, an insertion speed of the electrode lead to be inserted, etc.). The insertion configuration information may be provided to a user associated with the electrode lead insertion procedure (e.g., by way of display device 318).

The operation may additionally or alternatively include selecting a type of electrode lead to be inserted during the electrode lead insertion procedure, such as based on the one or more insertion factors. In some examples, the selecting the type of electrode lead may be based on weighting the one or more insertion factors. Additionally or alternatively, the types of electrode leads to be inserted during the electrode lead insertion procedure may be prioritized, such as based on the one or more insertion factors. The selecting the type of electrode lead may further be based on a pre-operative audiogram of the patient.

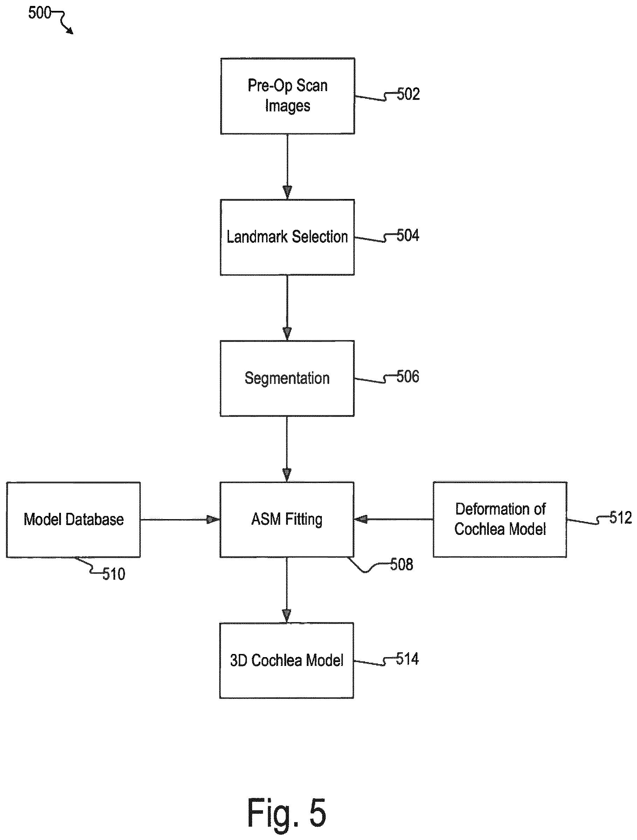

In certain examples, the accessing the 3D cochlea model may include generating an ASM-based cochlea model based on pre-operative scan images of the cochlea. In so doing, the shape and/or position of the scala tympani, scala vestibuli/scala media, and basilar membrane/osseous spiral lamina (BM/OSL) may be estimated from the pre-operative scan images. FIG. 5 illustrates an exemplary flow diagram 500 that includes various operations that may be performed by system 302 in generating an ASM-based 3D cochlea model. While FIG. 5 illustrates exemplary operations according to one embodiment, other embodiments may omit, add to, reorder, and/or modify any of the operations shown in FIG. 5.

As shown in FIG. 5, at operation 502, system 302 may access or otherwise obtain pre-operative scan images of the cochlea that depict the cochlea prior to insertion of the electrode lead in the cochlea.

At operation 504, system 302 may select landmarks for the cochlea within the pre-operative scan images. System 302 may select any suitable landmarks as may serve a particular implementation. In certain examples, the landmarks may be manually selected by, for example, a user such as a clinician. In certain alternative examples, the landmarks may be automatically selected by system 302 without requiring input from a user. For example, system 302 may implement any suitable machine learning algorithm or neural network algorithm to select the landmarks in certain implementations.

At operation 506, system 302 may perform a segmentation process. Such a segmentation process may be performed in any suitable manner. For example, system 302 may use a threshold-based segmentation that includes considering sharp transitions of hounsfield units. A hounsfield threshold may be selected by considering an area around the landmarks to provide good segmentation independent of the hounsfield range of the scan images.

At operation 508, system 302 may fit the ASM to the shape segmented at operation 506. An ASM of the cochlea wall, the basilar membrane, and the osseous spiral lamina may be used at operation 508. The basilar membrane and osseous spiral lamina may be represented by connecting two points, in each cross section, on the outer wall. The first point may be a projection of the spiral ligament on the lateral wall, the second point may be the intersection between the osseous spiral lamina and the medial wall. In this way, as the shape of the outer wall changes, so does the location of those two points and consequently the estimate of the basilar membrane and the osseous spiral lamina.

In certain examples, system 302 may use an ASM that is previously built based on a plurality of cochlea models segmented from high resolution scan images (e.g., high resolution CT scan images). As shown, system 302 may leverage a cochlea model database at operation 510 to facilitate fitting the ASM at operation 508. Such a cochlea model database may include information regarding a plurality of cochlea models that have been previously generated for other users. Cochlea models in the cochlea model database may be described as a combination of the scala tympani, the scala vestibuli, and the basilar membrane/osseous spiral lamina, and as an open duct. The cochlea models may be described till about the 600° depth of the cochlea. This may facilitate higher precision in the basal turn. The apical end of the cochlea may not be relevant for the cochlea implant and may complicate the fitting process. As such, the apical end of the cochlea may not be considered in certain examples when fitting the ASM.

System 302 may fit the ASM to the segmented shape in any suitable manner. For example, for the fitting, the outer wall of the segmented cochlea as well as the selected landmarks may be used. Thus, the fitting process may correspond to a point and surface-based process. Coherent point drifting (CPD), gaussian process regression (GPR) and regularized Laplacian deformation may be used to fit the ASM and select the right variations of shapes to lower the difference from segmentation to the ASM.

In certain examples, system 302 may deform a cochlea model at operation 512 to facilitate fitting the ASM at operation 508. This may be accomplished in any suitable manner. For example, system 302 may use regularized Laplacian deformation in certain examples. The deformation of the cochlea model may result in an estimation of the anatomical structure of the cochlea for the patient.

At operation 514, system 302 may output an ASM-based 3D cochlea model, which may be used in any suitable manner described herein such as to determine an insertion configuration of an electrode lead for an electrode lead insertion procedure.

Although flow diagram 500 shows a process where an ASM-based 3D cochlea model is generated based on pre-operative scan images, it is understood that ASM-based 3D cochlea models may be generated in other ways in certain alternative implementations.

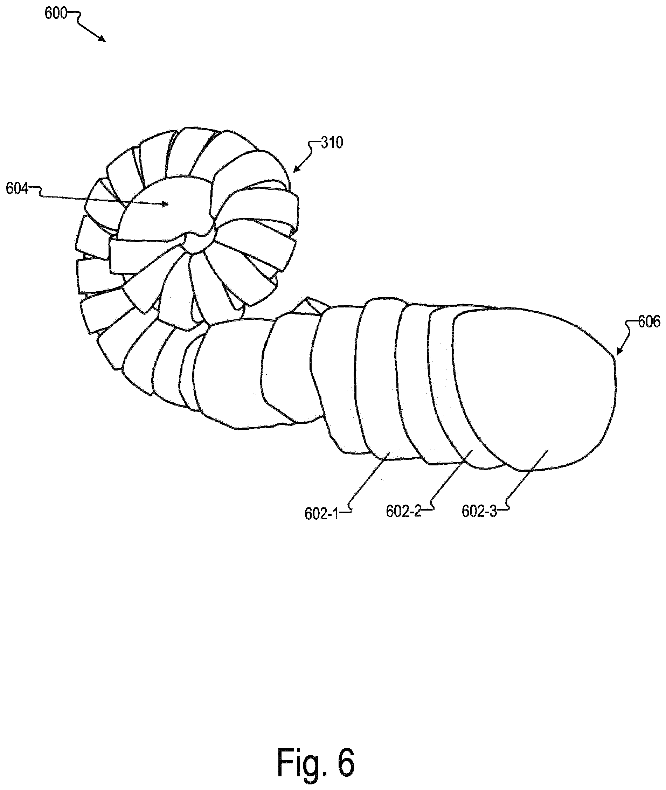

FIG. 6 shows an illustrative implementation 600 of an ASM-based 3D cochlea model (e.g., 3D cochlea model 310) that may be used for simulation as described herein. As shown, implementation 600 includes a plurality of segments 602 (e.g., segments 602-1, 602-2, 602-3, etc.) that extend from an apex portion 604 of the cochlea model to a portion 606 of the cochlea model at the round window. For example, system 302 may slice an ASM-based 3D cochlea model into the plurality of segments 602 ordered from the apex portion 604 of the cochlea to portion 606 at the round window of the cochlea. System 302 may slice the ASM-based 3D cochlea model into any suitable number of segments 602 as may serve a particular implementation. For example, system 302 may slice the ASM-based 3D cochlea model into five segments, ten segments, fifteen segments, twenty segments, or more than twenty segments. In certain examples, each segment 602 may extend a predefined distance along a length of the ASM-based 3D cochlea model. For example, each segment 602 may extend 1.5mm along a length of the ASM-based 3D cochlea model. In certain alternative examples, at least some segments 602 may extend different lengths along the ASM-based 3D cochlea model and/or may have different shapes.

In certain examples, the accessing the 3D electrode lead model may include generating the 3D electrode lead model such as based on images and/or known properties of the electrode lead. In so doing, the shape and/or position of the electrode lead may be estimated. To generate the 3D electrode lead model, system 302 may access or otherwise obtain images and/or known properties of the electrode lead prior to insertion of the electrode lead in the cochlea. System 302 may select landmarks for the electrode lead within the images. System 302 may select any suitable landmarks (e.g., a distal tip, electrodes, etc.) as may serve a particular implementation. In certain examples, the landmarks may be manually selected by, for example, a user such as a clinician. In certain alternative examples, the landmarks may be automatically selected by system 302 without requiring input from a user. For example, system 302 may implement any suitable machine learning algorithm or neural network algorithm to select the landmarks in certain implementations. In some examples, system 302 may segment the electrode lead (e.g., at areas around the landmarks). System 302 may further fit the model to the segmented shape.

In certain examples, system 302 may use an electrode lead model that is previously built based on a plurality of electrode lead models segmented from high resolution images. For example, system 302 may leverage an electrode lead model database to facilitate fitting the model. Such an electrode lead model database may include information regarding a plurality of electrode lead models that have been previously generated.

System 302 may fit the model to the segmented shape in any suitable manner. For example, for the fitting, the outer boundary of the electrode lead as well as the selected landmarks may be used. Thus, the fitting process may correspond to a point and surface-based process. Coherent point drifting (CPD), gaussian process regression (GPR) and regularized Laplacian deformation may be used to fit the ASM and select the right variations of shapes to lower the difference from segmentation to the model.

In certain examples, system 302 may deform an electrode lead model to facilitate fitting the model. This may be accomplished in any suitable manner. For example, system 302 may use regularized Laplacian deformation in certain examples. The deformation of the electrode lead model may result in an estimation of the structure of the electrode lead. System 302 may output a 3D electrode lead model, which may be used in any suitable manner described herein such as to determine an insertion configuration of the electrode lead for an electrode lead insertion procedure.

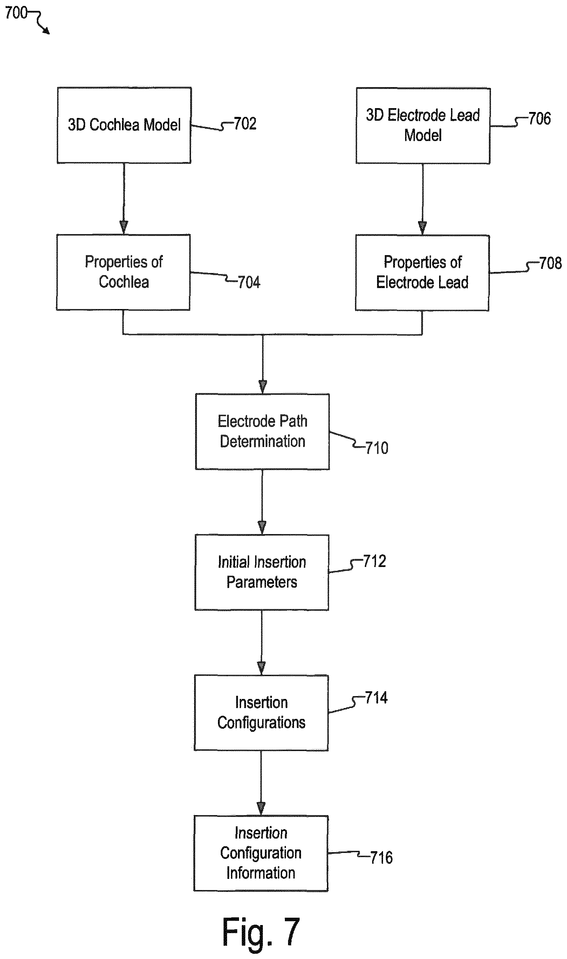

FIG. 7 depicts a flow diagram 700 with illustrative operations that may be performed by system 302 to perform a simulation using 3D cochlea model 310 and 3D electrode lead model 312. While FIG. 7 illustrates exemplary operations according to one embodiment, other embodiments may omit, add to, reorder, and/or modify any of the operations shown in FIG. 7.

As shown, at operation 702, system 302 may access a 3D cochlea model (e.g., 3D cochlea model 310). At operation 704, system 302 may determine one or more properties of the cochlea included in the 3D cochlea model based on the 3D cochlea model and/or pre-operative scan images. The one or more properties of the cochlea may include, but is not limited to, one or more characteristics (e.g., a size, a shape, a volume, a circumference, a diameter, a length, etc.) associated with anatomy (e.g., an apex portion, a round window, a scala tympani, a lateral wall, a mid-modiolar curve, a modiolar axis, a basilar membrane, a modiolar wall, an osseous spiral lamina, etc.) of the cochlea. System 302 may determine the one or more characteristics in any suitable manner. For example, system 302 may use any suitable 3D position and/or orientation information associated with the 3D cochlea model and/or pre-operative scan images to define boundaries associated with the anatomy of the cochlea. System 302 may determine any suitable number of characteristics as may serve a particular implementation.

At operation 706, system 302 may access a 3D electrode lead model (e.g., 3D electrode lead model 312). At operation 708, system 302 may determine one or more properties of an electrode lead (e.g., electrode lead 104) included in the 3D electrode lead model. The one or more properties of the electrode lead may include, but is not limited to, one or more physical properties (e.g., a type, a shape, a size, a stiffness, a material, an elastic modulus, a shear stress, a friction, etc.) associated with the electrode lead. In some examples, the one or more properties may further include points of interest associated with the electrode lead, such as a distal tip configured to be inserted within the cochlea and/or a position of one or more electrodes (e.g., electrodes 106) on the electrode lead. System 302 may determine the one or more properties in any suitable manner. For example, system 302 may use any suitable 3D position and/or orientation information associated with the 3D electrode lead model and/or images of the electrode lead to define boundaries associated with the electrode lead. System 302 may determine any suitable number of properties as may serve a particular implementation.

At operation 710, system 302 may determine an estimated electrode path for insertion of the electrode lead included in the 3D electrode lead model within the cochlea included in the 3D cochlea model. This may be accomplished in any suitable manner. For example, system 302 may slice the 3D cochlea model into a plurality of segments (e.g., segments 602) ordered from the apex of the cochlea to the round window of the cochlea. System 302 may slice the 3D cochlea model into any suitable number of segments as may serve a particular implementation. Based on the characteristics of the cochlea and/or the properties of the electrode lead, system 302 may analyze each segment to determine an estimated electrode path for insertion of the electrode lead within the cochlea from the round window to the apex portion.

At operation 712, system 302 may determine one or more initial insertion parameters associated with the insertion of the electrode lead included in the 3D electrode lead model within the cochlea included in the 3D cochlea model. The one or more initial insertion parameters may include, but is not limited to, one or more of a rotation, an insertion depth, an insertion angle, or an insertion speed of the electrode lead. System 302 may determine the one or more initial insertion parameters based on the characteristics of the cochlea, the properties of the electrode lead, a user input, and/or previous insertion parameters (e.g., insertion parameters previously and/or typically used for the electrode lead).

As an illustrative example, system 302 may determine an initial insertion angle of the electrode lead such as by determining a trajectory from the round window of the cochlea included in the cochlea model to a modiolar wall of the cochlea (e.g., between a BM/OSL of the cochlea and a bottom portion of the ST of the cochlea). System 302 may additionally determine an initial insertion depth of the electrode lead at a final placement such as by determining a length at which the electrode lead is positioned within the cochlea to provide sufficient placement of the plurality of electrodes of the electrode lead relative to the cochlea. In instances where the electrode lead includes a pre-curved electrode lead, the insertion depth may additionally or alternatively be determined by registering an axis of the pre-curved electrode lead with an axis of the cochlea.

At operation 714, system 302 may perform a simulation of an insertion configuration of the electrode lead included in the 3D electrode lead model within the cochlea included in the 3D cochlea model such as according to the one or more initial insertion parameters. For example, the distal tip of the electrode lead may be inserted through the round window of the cochlea with the electrode lead positioned at the initial insertion angle relative to the cochlea. The electrode lead may then be moved within the cochlea along the electrode path, such as at an initial speed and/or an initial rotation, to an initial insertion depth of the electrode lead at the final placement within the cochlea.

During the simulation, system 302 may determine a trauma value indicative of a trauma (e.g., damage to the inner structure of the cochlea, frictional stress to an outer wall of the cochlea, deflection of the basilar membrane of the cochlea, etc.) associated with the insertion of the electrode lead within the cochlea. The trauma value may be represented by a discrete value (e.g., a number, a range, a percentage, a level, etc.) and, in some instances, may be plotted relative to an insertion depth of the electrode lead. Accordingly, the trauma value may indicate trauma to the cochlea that may be induced from the insertion of the electrode lead within the cochlea.

System 302 may perform one or more additional simulations of additional one or more insertion configurations. In some examples, one or more insertion parameters may be adjusted and operations 710-714 may be repeated using the same electrode lead model. Additionally or alternatively, operations 706-714 may be repeated such that system 302 may access another electrode lead model (e.g., representative of another electrode lead having one or more different properties) and perform one or more simulations using the other electrode lead model with various insertion parameters.

At operation 716, system 302 may provide insertion configuration information (e.g., insertion configuration information 314). The providing the insertion configuration information may include analyzing simulations associated with each insertion configuration and selecting an insertion configuration and/or prioritizing the insertion configurations based on the simulations. For example, an insertion configuration may be selected that produced a minimum amount of trauma to the cochlea (e.g., relative to another insertion configuration) while being inserted and/or that provides a sufficient placement of the plurality of electrodes of the electrode lead within the cochlea.

As an illustrative example, one or more trauma values determined during simulation may indicate an increase of trauma to the cochlea at particular insertion depth and/or range of insertion depths of the electrode lead (e.g., a frictional stress to the lateral wall of the cochlea may increase at about 10-15 mm of insertion depth). As such, the selected insertion configuration may include a decrease in insertion speed and/or a rotation of the electrode lead prior to or at the particular insertion depth and/or range of insertion depths such as to decrease the amount of trauma to the cochlea during insertion of the electrode lead. Additionally or alternatively, the selected insertion configuration may include a type of electrode lead having an increased elasticity such as to decrease the amount of trauma to the cochlear during insertion of the electrode lead.

Based on the selected insertion configuration, information associated with the selected insertion configuration may be provided to a user (e.g., by way of display device 318). The information may include the one or more insertion parameters associated with the selected insertion configuration, which may be used for an electrode lead insertion procedure. Accordingly, the insertion configuration information may allow the electrode lead insertion procedure to be performed with a reduced or limited amount of trauma to the cochlea, an improved placement of the electrode lead and/or be performed more efficiently.

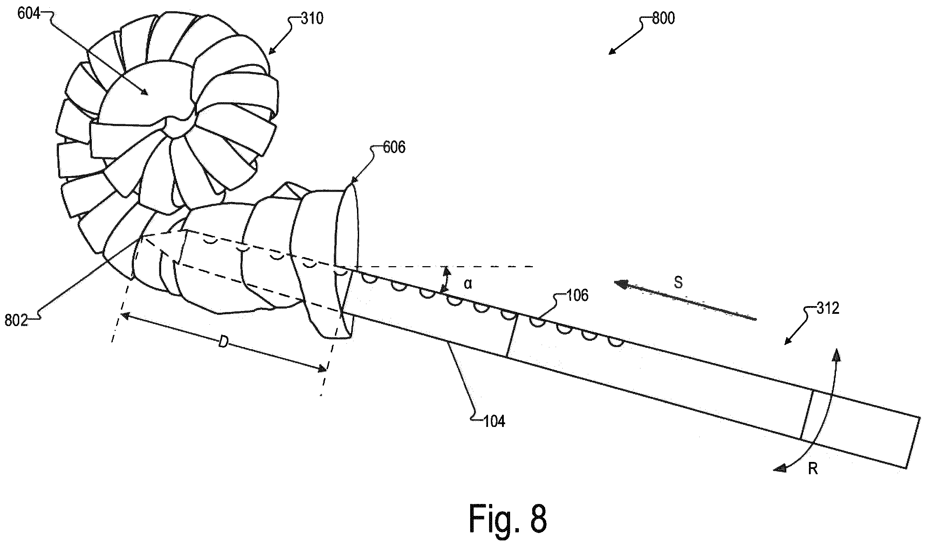

FIG. 8 shows an illustrative implementation 800 of a simulation of a 3D electrode lead model 312 being inserted within a 3D cochlea model 310. Electrode lead model 312 may be representative of an electrode lead 104 having a plurality of electrodes 106 positioned along electrode lead 104 from a distal tip 802 of electrode lead 104. The simulation is shown as being performed according to one or more insertion parameters. For example, distal tip 802 of 3D electrode lead model 312 has been inserted through portion 606 at the round window of 3D cochlea model 310 to an insertion depth D. Electrode lead model 312 is further being inserted at an insertion angle α relative to the cochlea (e.g., at portion 606). During the simulation, 3D electrode lead model 312 may be moved further within 3D cochlea model 310, such as at an insertion speed S and/or at an insertion rotation R (e.g., in a direction clockwise and/or counterclockwise relative to portion 606). Still other suitable insertion parameters may be used (e.g., a size, shape, and/or type of electrode lead 104).

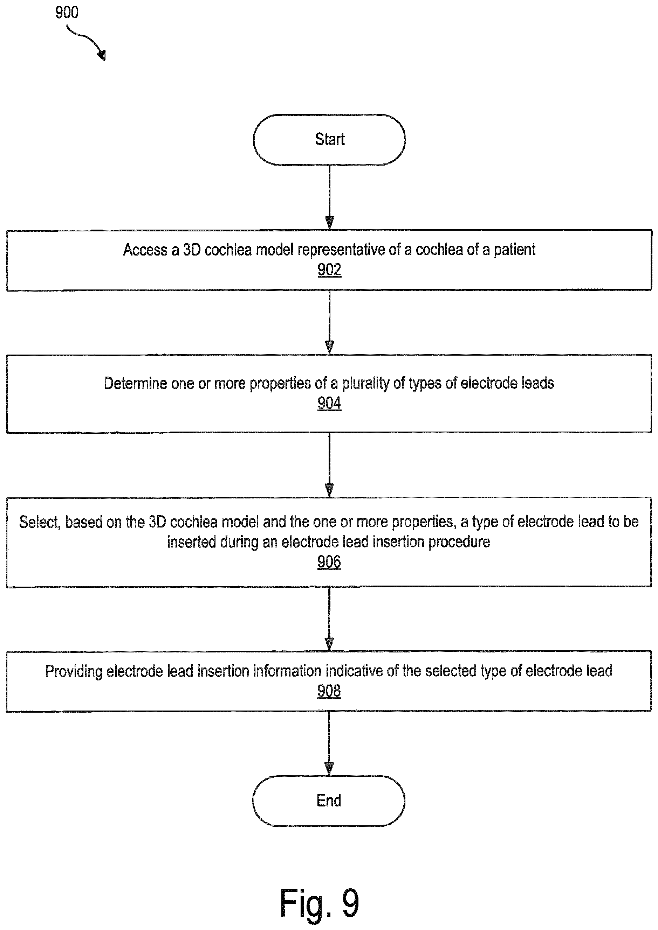

FIG. 9 shows an illustrative method 900 for selecting an electrode lead for an electrode lead insertion procedure. While FIG. 9 illustrates exemplary operations according to one embodiment, other embodiments may omit, add to, reorder, and/or modify any of the operations shown in FIG. 9. Moreover, each of the operations depicted in Fig. 9 may be performed in any of the ways described herein. One or more of the operations depicted in Fig. 9 may be performed by cochlear implant system 100 and/or system 302.

Method 900 may include, at operation 902, accessing a 3D cochlea model (e.g., 3D cochlea model 310) representative of a cochlea of a patient within which an electrode lead (e.g., electrode lead 104) included in a cochlear implant system (e.g., cochlear implant system 100) is to be inserted during an electrode lead insertion procedure.

Method 900 may further include, at operation 904, determining one or more properties of a plurality of types of electrode leads (e.g., perimodiolar, lateral wall, mid-scala, straight, pre-curved, etc.) configured to be inserted within the cochlea. The one or more properties may include one or more physical properties (e.g., a type, a shape, a size, a stiffness, a material, an elastic modulus, a shear stress, a friction, etc.) for each electrode lead included in the plurality of types of electrode leads. In some examples, the one or more properties may be based on one or more 3D electrode lead models (e.g., 3D electrode lead model 312) and/or images of the electrode leads.

Method 900 may further include, at operation 906, selecting, based on the 3D cochlea model and the one or more properties, a type of electrode lead included in the plurality of types of electrode leads to be inserted during the electrode lead insertion procedure. To illustrate, the type of electrode lead may be selected by comparing the one or more properties of the types of electrode leads to the 3D cochlea model such as to determine a type of electrode lead that may best fit the cochlea included in the 3D cochlea model and/or be inserted with a minimum amount of trauma to the cochlea (e.g., relative to another type of electrode lead). As an illustrative example, a length of a type of electrode lead may be compared to a length of a cochlea duct of the 3D cochlea model such as to determine a type of cochlea lead having a length that corresponds to the length of the cochlea duct. Additionally or alternatively, a radius of a pre-curved electrode lead may be compared to a radius of a mid-modiolar curve of the 3D cochlea model such as to determine a type of electrode lead having a radius that corresponds to the mid-modular curve. Still other suitable configurations may be used to select the type of electrode lead.

For example, the selecting the type of electrode lead may include simulating one or more insertion configurations associated with insertion of one or more electrode leads included in the plurality of types of electrode leads within the 3D cochlea model. The simulating may use one or more 3D electrode lead models (e.g., 3D electrode lead model 312) representative of one or more electrode leads included in the plurality of types of electrode leads configured to be inserted within the cochlea. Such one or more 3D electrode lead models may include the one or more properties of the plurality of types of electrode leads. Moreover, each insertion configuration of the one or more insertion configurations may include one or more insertion parameters, such as one or more of a type of one or more electrode leads, a rotation of one or more electrode leads, an insertion depth of one or more electrode leads, an insertion angle of one or more electrode leads, or an insertion speed of one or more electrode leads. The simulating the one or more insertion configurations may further include varying the one or more insertion parameters during the simulating (e.g., during a simulation and/or between simulations).

In some examples, the selecting the type of electrode lead may further be based on one or more insertion factors, such as one or more of an insertion angle of each of the types of electrode leads, an anatomy of the cochlea (e.g., an ST height, a cochlea duct length, a cochlear radius, etc.), or a proximity of a facial nerve to the cochlea. The one or more insertion factors may further be weighted to select the type of electrode lead. Additionally or alternatively, the one or more insertion factors may be compared to a predetermined threshold for selecting the type of electrode lead. Moreover, the selecting the type of electrode lead and/or determining the one or more insertion factors may be based on a pre-operative audiogram of the patient.

Method 900 may further include, at operation 908, providing electrode lead insertion information indicative of the type of electrode lead to be inserted during the electrode lead insertion procedure. The electrode lead insertion information may include one or more of the type of the electrode lead to be inserted, a size of the electrode lead to be inserted, a rotation of the electrode lead to be inserted, an insertion depth of the electrode lead to be inserted, an insertion angle of the electrode lead to be inserted, or an insertion speed of the electrode lead to be inserted. Such electrode lead insertion information may be provided to a display device (e.g., display device 318) such as for display to a user associated with the electrode lead insertion procedure. Such selection of a type of electrode lead for the electrode lead insertion procedure may reduce or limit trauma to the cochlea, improve placement of the electrode lead, and/or improve an efficiency of the electrode lead insertion procedure.

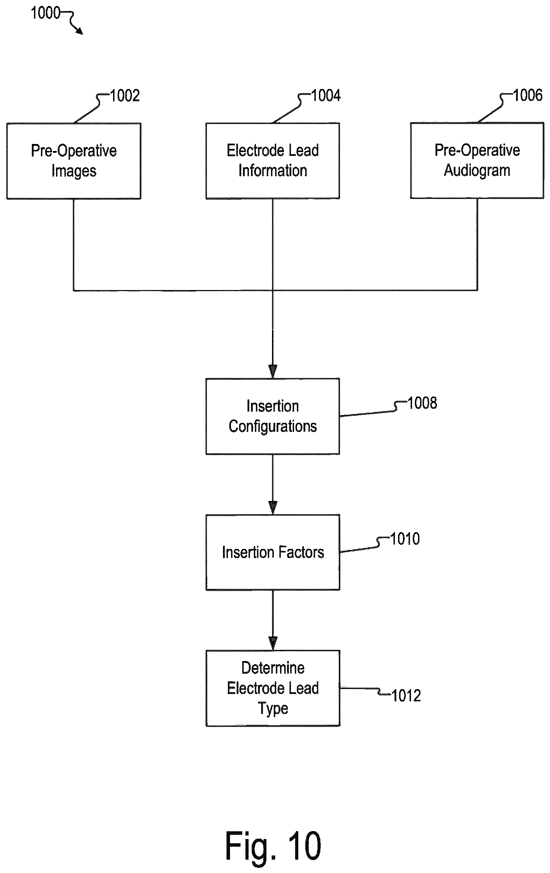

FIG. 10 depicts an illustrative flow diagram 1000 with operations that may be performed by system 302 to determine a type of electrode lead to be inserted during an electrode lead insertion procedure. While FIG. 10 illustrates exemplary operations according to one embodiment, other embodiments may omit, add to, reorder, and/or modify any of the operations shown in FIG. 10.

As shown, at operation 1002, system 302 may access pre-operative images of a cochlea of a patient within which an electrode lead is to be inserted during the electrode lead insertion procedure. In some examples, the accessing the pre-operative images may include accessing a 3D cochlea model (e.g., 3D cochlea model 310) representative of the cochlea. In such examples, the 3D cochlea model may be generated based on the pre-operative images of the cochlea.

At operation 1004, system 302 may access electrode lead information associated with a plurality of types of electrode leads configured to be inserted within the cochlea. The electrode lead information may include one or more properties of the plurality of types of electrode leads, such as a type (e.g., perimodiolar, lateral wall, mid-scala, straight, pre-curved, etc.) of electrode lead and/or one or more physical properties (e.g., a type, a shape, a size, a stiffness, a material, an elastic modulus, a shear stress, a friction, etc.) for each type of electrode lead included in the plurality of types of electrode leads. In some examples, the one or more properties may be based on one or more 3D electrode lead models (e.g., 3D electrode lead model 312) and/or images of the electrode leads.

At operation 1006, system 302 may access data representative of a pre-operative audiogram of the patient. The data may be indicative of an amplitude of sounds that the patient may hear at various frequencies. The data may further be indicative of a type, a degree, and/or a configuration of hearing loss associated with the patient. In some examples, system 302 may further access data representative of one or more objective measures (e.g., eABR, ECAPS, EcochG, etc.) associated with the patient.

At operation 1008, system 302 may determine one or more insertion configurations associated with the plurality of types of electrode leads. For example, system 302 may simulate one or more insertion configurations associated with insertion of one or more electrode leads included in the plurality of types of electrode leads within the 3D cochlea model. The simulating may use one or more 3D electrode lead models (e.g., 3D electrode lead model 312) representative of one or more electrode leads included in the plurality of types of electrode leads configured to be inserted within the cochlea. Such one or more 3D electrode lead models may include the one or more properties of the plurality of types of electrode leads. Moreover, each insertion configuration of the one or more insertion configurations may include one or more insertion parameters, such as one or more of a type of one or more electrode leads, a rotation of one or more electrode leads, an insertion depth of one or more electrode leads, an insertion angle of one or more electrode leads, or an insertion speed of one or more electrode leads. The simulating the one or more insertion configurations may further include varying the one or more insertion parameters during the simulating (e.g., during a simulation and/or between simulations). Alternatively, the one or more insertion configurations and/or insertion parameters may be determined based on the pre-operative images, the electrode lead information, and/or the pre-operative audiogram data without simulating the one or more insertion configurations.

At operation 1010, system 302 may determine one or more insertion factors associated with the plurality of types of electrode leads. The one or more insertion factors may include one or more of an insertion angle of each of the types of electrode leads, an anatomy of the cochlea (e.g., an ST height, a cochlea duct length, a cochlear radius, etc.), or a proximity of a facial nerve to a position (e.g., at a 270° of angular depth from the round window) of each type of electrode lead. The one or more insertion factors may be determined based on the pre-operative images, the electrode lead information, and/or the pre-operative audiogram data. To illustrate, an insertion angle of a type of electrode lead may be determined based on the pre-operative images such as by determining a trajectory from the round window to the mid-modiolar wall. An ST height may be determined based on the pre-operative images, the electrode lead information (e.g., electrode lead dimensions), and/or the pre-operative audiogram. A cochlear duct length may be determined based on the pre-operative images and/or the pre-operative audiogram. The cochlear radius and/or facial nerve proximity to the cochlea may be determined based on the pre-operative images, such as by estimating the radius and/or the distance of the of facial nerve canal to inner cochlea wall based on the pre-operative images. The determining the one or more insertion factors may further include weighting the one or more insertion factors. Additionally or alternatively, the one or more insertion factors may be compared to a predetermined threshold associated with the one or more insertion factors.

At operation 1012, system 302 may determine the type of electrode lead included in the plurality of types of electrode leads to be inserted during the electrode lead insertion procedure. The type of electrode lead may be determined based on the one or more insertion configurations and/or one or more insertion factors. For example, a type of electrode lead having an insertion configuration that reduces trauma to the cochlea may be selected. Additionally or alternatively, a type of electrode lead may be selected that corresponds to the one or more insertion factors.

To illustrate, straight electrode leads generally depend on the individual cochlear anatomy of the patient, whereas pre-curved electrode leads are more generally independent of the cochlear anatomy due to their pre-curved form. In instances where a particular insertion depth and/or a consistent insertion depth for a particular patient is selected, a pre-curved electrode lead may be selected.

Moreover, intracochlear dimensions of the scala tympany may vary between individuals from the round window towards the apex of the cochlea. For example, the height along the lateral wall of the scala tympani may decrease more quickly as compared to the height at the center or modiolar position. Depending on the available height of the scala tympani for the individual patient, a straight or a pre-curved electrode lead may be selected to achieve an atraumatic insertion with optimal performance outcomes. Additionally, in instances where the width of the cochlea is narrow, a lateral wall electrode lead may be forced to bend above a critical point such that a pre-curved electrode may be selected to reduce strain on the electrode lead.

The facial nerve proximity may vary between individuals such that, in some cases, the facial nerve canal may be close to the cochlear wall. This close proximity may lead to facial nerve stimulation during operation of the cochlear implant system such that a pre-curved electrode lead may be selected to position the electrode lead farther away from the facial nerve as compared to a straight electrode lead. In some examples, the distance between the facial nerve and the cochlea wall may be compared to a predetermined threshold and the selecting the type of electrode lead may be based on whether the distance is below the predetermined threshold.

As another example, the position of the facial nerve and/or chorda tympani may obstruct the accessibility of the cochlea. Moreover, based on the insertion angle, an insertion trajectory may turn sharply after entering the cochlea towards the modiolar wall. In such instances, a more flexible straight electrode lead may be selected to allow for a more atraumatic insertion and/or a lateral wall electrode lead may be selected to allow for an easier insertion.

Moreover, the type of electrode lead may be selected based on the final placement of the electrode lead such as to increase performance of the cochlear implant system for the patient. To illustrate, the final placement of the electrode lead may provide coverage of spiral ganglion cells within the cochlea. One or more objective measures may indicate a survival rate of the spiral ganglion cells, as some regions along the cochlea may have degenerated spiral ganglion cells (e.g., dead regions). In instances where dead regions are present, pre-curved electrode leads located closer to the nerve fibers may have a poorer performance due to reduced current spread. Accordingly, a straight electrode lead may be selected to bridge the dead regions due to the wider current spread. Alternatively, in instances where dead regions are not present, a pre-curved electrode lead may be selected to lead to an increased performance due to the reduced current spread.

The determining the type of electrode lead may further include providing electrode lead insertion information indicative of the selected type of electrode lead to be inserted during the electrode lead insertion procedure. The electrode lead insertion information may include one or more of the type of the electrode lead to be inserted, a size of the electrode lead to be inserted, a rotation of the electrode lead to be inserted, an insertion depth of the electrode lead to be inserted, an insertion angle of the electrode lead to be inserted, or an insertion speed of the electrode lead to be inserted. Such electrode lead insertion information may be provided to a display device (e.g., display device 318) such as for display to a user associated with the electrode lead insertion procedure. Such selection of a type of electrode lead for the electrode lead insertion procedure may reduce or limit trauma to the cochlea, improve placement of the electrode lead, and/or improve an efficiency of the electrode lead insertion procedure.

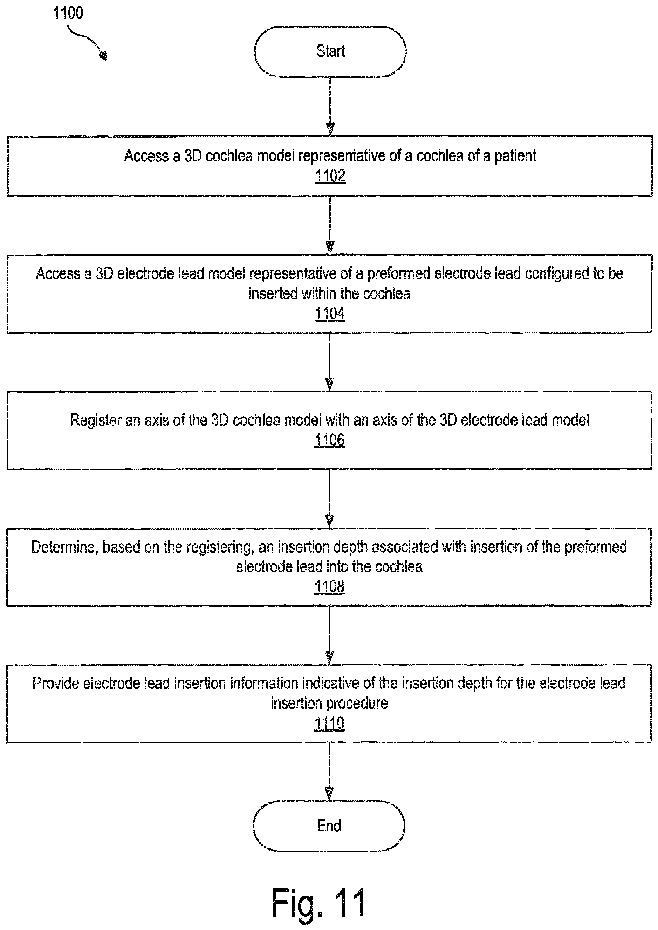

FIG. 11 illustrates an illustrative method 1100 for determining an insertion depth of an electrode lead to be inserted during an electrode lead insertion procedure. While FIG. 11 illustrates exemplary operations according to one embodiment, other embodiments may omit, add to, reorder, and/or modify any of the operations shown in FIG. 11. Moreover, each of the operations depicted in Fig. 11 may be performed in any of the ways described herein. One or more of the operations depicted in Fig. 11 may be performed by cochlear implant system 100 and/or system 302.

Method 1100 may include, at operation 1102, accessing a 3D cochlea model (e.g., 3D cochlea model 310) representative of a cochlea of a patient within which an electrode lead included in a cochlear implant system is to be inserted during an electrode lead insertion procedure. In some examples, the accessing the 3D cochlea model may further include identifying one or more points of interest of the cochlea included in the 3D cochlea model, such as one or more of a round window of the cochlea, a mid-modiolar wall of the cochlea, a mid-modiolar curve of the cochlea (e.g., defined by a center of the BM/OSL), or an axis about which the mid-modular curve is positioned.

Method 1100 may further include, at operation 1104, accessing an 3D electrode lead model (e.g., 3D electrode lead model 312) representative of a preformed (e.g., pre-curved) electrode lead configured to be inserted within the cochlea. The 3D electrode lead model may include one or more properties of the preformed electrode lead, such as a stiffness associated with the preformed electrode lead, a material associated with the preformed electrode lead, a size associated with the preformed electrode lead, an elastic modulus associated with the preformed electrode lead, a shear stress associated with the preformed electrode lead, and/or a friction associated with the preformed electrode lead. In some examples, the accessing the 3D electrode lead model may further include identifying one or more points of interest of the electrode lead included in the 3D electrode lead model, such as one or more of a distal tip of the electrode lead, one or more markers associated with the electrode lead, or an axis about which the preformed electrode lead is curved.

Method 1100 may further include, at operation 1106, registering an axis of the 3D cochlea model with an axis of the 3D electrode lead model. For example, the axis associated with the mid-modiolar curve of the cochlea may be registered with the axis associated with the curve of the preformed electrode lead such as to align the axis of the 3D cochlea model with the axis of the 3D electrode lead model. In some examples, the registering the axis of the 3D cochlea model with the axis of the 3D electrode lead model may further include rotating the 3D electrode lead model such as until a basal portion of the 3D electrode lead model contacts the round window.

Method 1100 may further include, at operation 1108, determining, based on the registering, an insertion depth associated with insertion of the preformed electrode lead into the cochlea. In some examples, the determining the insertion depth may include determining a first insertion depth of the preformed electrode lead at which the preformed electrode lead is released from a stylet or sheath. As such, the determining the first insertion depth may include determining a trajectory from the round window of the cochlea included in the cochlea model to a modiolar wall of the cochlea (e.g., between a BM/OSL of the cochlea and a bottom portion of the ST of the cochlea) such as to determine the insertion angle of the preformed electrode lead relative to the cochlea (e.g., the round window). Based on the insertion angle, a distance from the round window to the mid-modiolar curve of the cochlea along the trajectory (e.g., where the trajectory intersects the mid-modiolar curve) may be determined, which may correspond to the first insertion depth. In some instances, the preformed electrode lead may include a first marker such that the first insertion depth is provided relative to the first marker.

Additionally or alternatively, the determining the insertion depth may include determining a second insertion depth of the preformed electrode lead at which the preformed electrode lead is fully inserted within the cochlea. For example, based on the position of the axis of the preformed electrode lead being aligned with the axis of the cochlea, the preformed electrode may be rotated about the axis of the preformed electrode until a basal portion of the preformed electrode contacts the round window (e.g., such that the preformed electrode is positioned at or near the mid-modiolar curve of the cochlea). A depth of the preformed electrode within the cochlea at such a position may correspond to the second insertion depth. In some instances, the preformed electrode lead may include a second marker such that the second insertion depth is provided as a depth of the second marker relative to the round window of the cochlea.

In some examples, the determining the insertion depth may be further based on simulating one or more insertion configurations associated with insertion of the preformed electrode lead into the cochlea using the 3D cochlea model and the 3D electrode lead model. Each insertion configuration of the one or more insertion configurations may include one or more insertion parameters, such as one or more of a type of the preformed electrode lead, a rotation of the preformed electrode lead, an insertion depth of the preformed electrode lead, an insertion angle of the preformed electrode lead, and/or an insertion speed of the preformed electrode lead. The simulating the one or more insertion configurations may include varying the one or more insertion parameters during the simulating.

Method 1100 may further include, at operation 1110, providing electrode lead insertion information indicative of the insertion depth for the electrode lead insertion procedure. The electrode lead insertion information may further include one or more of a type of the preformed electrode lead to be inserted, a size of the preformed electrode lead to be inserted, a rotation of the preformed electrode lead to be inserted, an insertion angle of the preformed electrode lead to be inserted, or an insertion speed of the preformed electrode lead to be inserted. Such electrode lead insertion information may be provided to a display device (e.g., display device 318) such as for display to a user associated with the electrode lead insertion procedure. Such determination of insertion depth for the electrode lead insertion procedure may reduce or limit trauma to the cochlea, improve placement of the electrode lead, and/or improve an efficiency of the electrode lead insertion procedure.

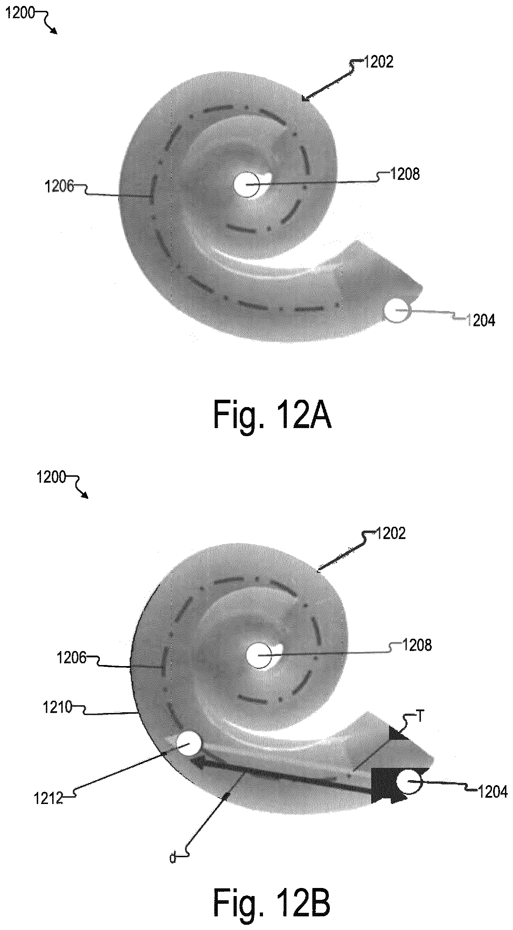

As an illustrative example, FIGS. 12A-12B show an implementation 1200 for determining a first insertion depth of a preformed electrode lead at which the preformed electrode lead may be released from a stylet or sheath. Accordingly, FIG. 12A shows a 3D cochlea model 1202 that may implement or be similar to 3D cochlea model 310. 3D cochlea model 1202 includes a round window 1204 and a mid-modiolar curve 1206 positioned about a mid-modiolar axis 1208. FIG. 12B further shows that 3D cochlea model 1202 includes a trajectory T from round window 1204 to a modiolar wall 1210 of 3D cochlea model 1202. This trajectory T may be used to determine the insertion angle of the preformed electrode lead relative to the 3D cochlea model 1202. Additionally, trajectory T may be used to determine an intersection point 1212 of 3D cochlea model 1202 at the intersection of mid-modiolar curve 1206 and the trajectory T. A distance d from round window 1204 to intersection point 1212 may then be determined as the first insertion depth of the preformed electrode lead for the electrode lead insertion procedure. Still other suitable configurations may be used for determining the insertion depth.

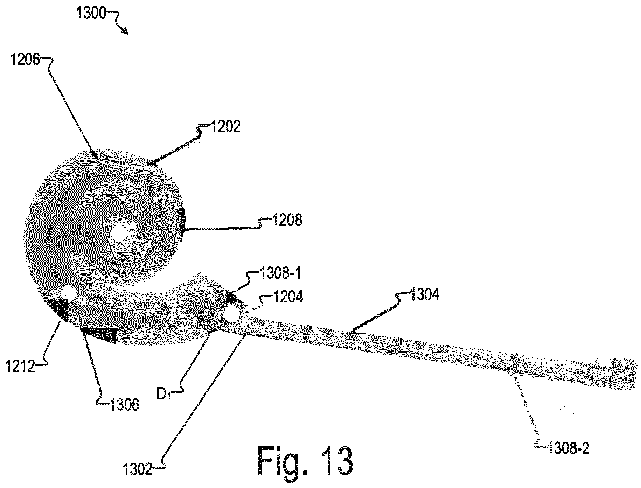

For example, the first insertion depth may be determined based on a marker associated with the preformed electrode lead. To illustrate, FIG. 13 shows an implementation 1300 including a 3D electrode lead model 1302 inserted within 3D cochlea model 1202. 3D electrode lead model 1302 may implement to be similar to electrode lead model 312. As shown, 3D electrode lead model 1302 is representative of a preformed electrode lead including a plurality of electrodes 1304 positioned on electrode lead model 1302 from a distal tip 1306 of electrode lead model 1302 and one or more markers 1308 (e.g., markers 1308-1 through 1308-2). Markers 1308 may include any suitable visual marker (e.g., a line, an overlay, highlighting, etc.). The preformed electrode lead included in electrode lead model 1302 is configured to be inserted within the cochlea of 3D cochlea model 1202 such that distal tip 1306 of electrode lead model 1302 is inserted within the cochlea at round window 1204, along the trajectory T, to intersection point 1212.

Based on electrode lead model 1302 and cochlea model 1202, a size of the preformed electrode lead included in electrode lead model 1302 is known as well as the distance d from round window 1204 to intersection point 1212 of the cochlea such that a first relative distance D₁ between a first marker 1308-1 of the electrode lead and round window 1204 may be determined as the first insertion depth. The first relative distance D₁ may be determined in any direction relative to round window 1204 such that the first relative distance D₁ may correspond to first marker 1308-1 being inserted beyond round window 1204 or before round window 1204. Moreover, while the illustrated example shows first relative distance D₁ as being determined between first marker 1308-1 and round window 1204, any other suitable components of the cochlea may be used to determine the first relative distance D₁. Such a first insertion depth may be provided to a user for insertion of the preformed electrode lead during an electrode lead insertion procedure. For example, the preformed electrode lead may be inserted within the cochlea until first marker 1308-1 of the preformed electrode lead is positioned at the first relative distance D₁ from round window 1204, where the preformed electrode lead may be released from the stylet or sheath.

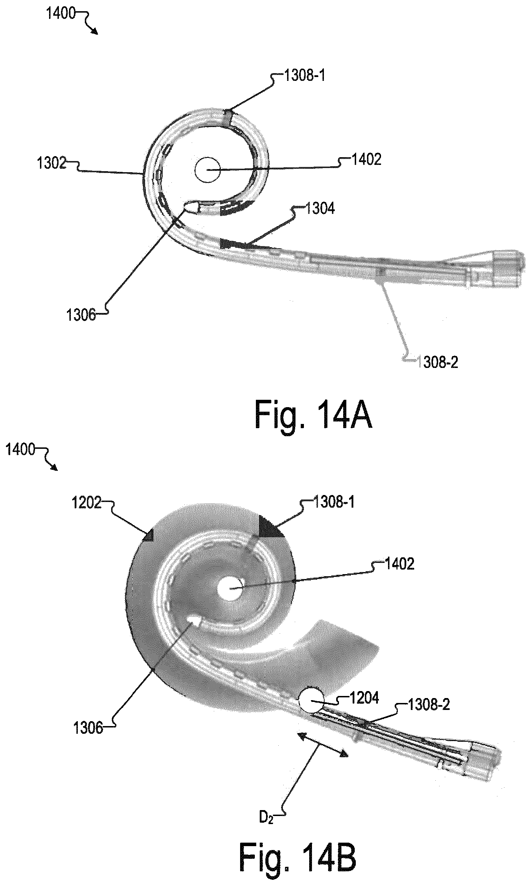

As another illustrative example, FIGS. 14A-14B an implementation 1400 of determining a second insertion depth of the preformed electrode lead at which the preformed electrode lead is fully inserted within the cochlea. FIG. 14A shows a final position of the preformed electrode lead included in electrode lead model 1302 after the preformed electrode lead has been released from the stylet or sheath. As shown, the preformed electrode lead is curved about an axis 1402. Accordingly, as shown in FIG. 14B, axis 1402 of electrode lead model 1302 may be registered with axis 1208 of cochlea model 1202 such as to align axis 1402 with axis 1208. The preformed electrode of electrode lead model 1302 may further be rotated about the axis 1402 of the preformed electrode until a basal portion of the preformed electrode contacts round window 1204 of the cochlea (e.g., such that the preformed electrode is positioned at or near the mid-modiolar curve of the cochlea).

A depth of the preformed electrode at such a position may correspond to the second insertion depth. Additionally or alternatively, 3D electrode lead model 1302 may include a second marker 1308-2 such that the second insertion depth may be determined as a second relative distance D₂ between the second marker 1308-2 and round window 1204 of the cochlea. The second relative distance D₂ may be determined in any direction relative from round window 1204 such that the second relative distance D₂ may correspond to second marker 1308-2 being inserted beyond round window 1204 or before round window 1204. Moreover, while the illustrated example shows second relative distance D₂ as being determined between second marker 1308-2 and round window 1204, any other suitable components of the cochlea may be used to determine the second relative distance D₂. Such a second insertion depth may be provided to a user for insertion of the preformed electrode lead during an electrode lead insertion procedure. For example, after the preformed electrode lead is released from the stylet or sheath, the preformed electrode lead may continue to be inserted within the cochlea until second marker 1308-2 of the preformed electrode lead is positioned at the second relative distance D₂ relative to round window 1204, where the preformed electrode lead may be positioned in a final placement within the cochlea.

Still other suitable configurations for inserting the preformed electrode lead within the cochlea may be used. For example, while the illustrated examples include determining relative distances in two-dimensions, the relative distances may further be determined in three-dimensions (e.g., relative to an ST height of the cochlea).

In some examples, a computer program product embodied in a non-transitory computer-readable storage medium may be provided. In such examples, the non-transitory computer-readable storage medium may store computer-readable instructions in accordance with the principles described herein. The instructions, when executed by a processor of a computing device, may direct the processor and/or computing device to perform one or more operations, including one or more of the operations described herein. Such instructions may be stored and/or transmitted using any of a variety of known computer-readable media.

A non-transitory computer-readable medium as referred to herein may include any non-transitory storage medium that participates in providing data (e.g., instructions) that may be read and/or executed by a computing device (e.g., by a processor of a computing device). For example, a non-transitory computer-readable medium may include, but is not limited to, any combination of non-volatile storage media and/or volatile storage media. Exemplary non-volatile storage media include, but are not limited to, read-only memory, flash memory, a solid-state drive, a magnetic storage device (e.g., a hard disk, a floppy disk, magnetic tape, etc.), ferroelectric random-access memory ("RAM"), and an optical disc (e.g., a compact disc, a digital video disc, a Blu-ray disc, etc.). Exemplary volatile storage media include, but are not limited to, RAM (e.g., dynamic RAM).

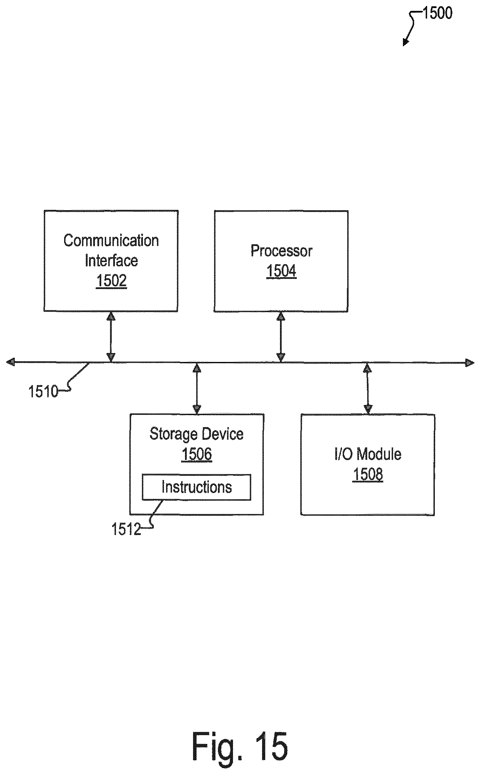

FIG. 15 illustrates an exemplary computing device 1500 that may be specifically configured to perform one or more of the processes described herein. As shown in FIG. 15, computing device 1500 may include a communication interface 1502, a processor 1504, a storage device 1506, and an input/output ("I/O") module 1508 communicatively connected one to another via a communication infrastructure 1510. While an exemplary computing device 1500 is shown in FIG. 15, the components illustrated in FIG. 15 are not intended to be limiting. Additional or alternative components may be used in other embodiments. Components of computing device 1500 shown in FIG. 15 will now be described in additional detail.

Communication interface 1502 may be configured to communicate with one or more computing devices. Examples of communication interface 1502 include, without limitation, a wired network interface (such as a network interface card), a wireless network interface (such as a wireless network interface card), a modem, an audio/video connection, and any other suitable interface.

Processor 1504 generally represents any type or form of processing unit capable of processing data and/or interpreting, executing, and/or directing execution of one or more of the instructions, processes, and/or operations described herein. Processor 1504 may perform operations by executing computer-executable instructions 1512 (e.g., an application, software, code, and/or other executable data instance) stored in storage device 1506.

Storage device 1506 may include one or more data storage media, devices, or configurations and may employ any type, form, and combination of data storage media and/or device. For example, storage device 1506 may include, but is not limited to, any combination of the non-volatile media and/or volatile media described herein. Electronic data, including data described herein, may be temporarily and/or permanently stored in storage device 1506. For example, data representative of computer-executable instructions 1512 configured to direct processor 1504 to perform any of the operations described herein may be stored within storage device 1506. In some examples, data may be arranged in one or more databases residing within storage device 1506.

I/O module 1508 may include one or more I/O modules configured to receive user input and provide user output. I/O module 1508 may include any hardware, firmware, software, or combination thereof supportive of input and output capabilities. For example, I/O module 1508 may include hardware and/or software for capturing user input, including, but not limited to, a keyboard or keypad, a touchscreen component (e.g., touchscreen display), a receiver (e.g., an RF or infrared receiver), motion sensors, and/or one or more input buttons.

I/O module 1508 may include one or more devices for presenting output to a user, including, but not limited to, a graphics engine, a display (e.g., a display screen), one or more output drivers (e.g., display drivers), one or more audio speakers, and one or more audio drivers. In certain embodiments, I/O module 1508 is configured to provide graphical data to a display for presentation to a user. The graphical data may be representative of one or more graphical user interfaces and/or any other graphical content as may serve a particular implementation.

In some examples, any of the systems, hearing devices, computing devices, and/or other components described herein may be implemented by computing device 1500. For example, memory 304 may be implemented by storage device 1506, and processor 306 may be implemented by processor 1504.

Advantages and features of the present disclosure can be further described by the following examples:
Example 1. A system comprising: a memory storing instructions; and a processor configured to execute the instructions to perform a process comprising: accessing a three-dimensional (3D) cochlea model representative of a cochlea of a patient within which an electrode lead included in a cochlear implant system is to be inserted during an electrode lead insertion procedure; accessing one or more 3D electrode lead models representative of one or more electrode leads configured to be inserted within the cochlea, the one or more 3D electrode lead models including one or more properties of the one or more electrode leads; simulating, based on the 3D cochlea model and the one or more 3D electrode lead models, one or more insertion configurations associated with insertion of the one or more electrode leads into the cochlea; and performing, based on the simulating, an operation associated with the electrode lead insertion procedure.
Example 2. The system of any of the preceding examples, wherein the 3D cochlea model is based on pre-operative imaging of the cochlea of the patient.
Example 3. The system of any of the preceding examples, wherein the one or more properties of the one or more electrode leads includes one or more of a stiffness associated with the one or more electrode leads, a material associated with the one or more electrode leads, a size associated with the one or more electrode leads, one or more elastic moduli associated with the one or more electrode leads, a shear stress associated with the one or more electrode leads, or a friction associated with the one or more electrode leads.
Example 4. The system of any of the preceding examples, wherein each insertion configuration of the one or more insertion configurations includes one or more insertion parameters.
Example 5. The system of any of the preceding examples, wherein the one or more insertion parameters include one or more of a type of the one or more electrode leads, a rotation of the one or more electrode leads, an insertion depth of the one or more electrode leads, an insertion angle of the one or more electrode leads, or an insertion speed of the one or more electrode leads.
Example 6. The system of any of the preceding examples, wherein the simulating the one or more insertion configurations includes varying the one or more insertion parameters during the simulating.
Example 7. The system of any of the preceding examples, wherein the one or more insertion parameters are designated based on a user input.
Example 8. The system of any of the preceding examples, wherein the simulating the one or more insertion configurations includes simulating trauma to the cochlea for each insertion configuration.
Example 9. The system of any of the preceding examples, wherein the performing the operation includes prioritizing the one or more insertion configurations based on reducing trauma to the cochlea such that a first insertion configuration associated with less trauma to the cochlea than a second insertion configuration is prioritized above the second insertion configuration.
Example 10. The system of any of the preceding examples, wherein the performing the operation includes selecting an insertion configuration of the one or more insertion configurations based on reducing trauma to the cochlea.
Example 11. The system of any of the preceding examples, wherein the performing the operation includes providing insertion configuration information for the electrode lead insertion procedure.
Example 12. The system of any of the preceding examples, wherein the insertion configuration information includes one or more of a type of the electrode lead to be inserted, a rotation of the electrode lead to be inserted, an insertion depth of the electrode lead to be inserted, an insertion angle of the electrode lead to be inserted, or an insertion speed of the electrode lead to be inserted.
Example 13. The system of any of the preceding examples, wherein the performing the operation includes selecting, based on one or more insertion factors, a type of electrode lead to be inserted during the electrode lead insertion procedure.
Example 14. The system of any of the preceding examples, wherein the one or more insertion factors includes one or more of an insertion angle of the electrode lead, an anatomy of the cochlea, or a proximity of a facial nerve to a position of the electrode lead.
Example 15. The system of any of the preceding examples, wherein the selecting the type of electrode lead includes weighting the one or more insertion factors.
Example 16. The system of any of the preceding examples, wherein the selecting the type of electrode lead is further based on a pre-operative audiogram of the patient.
Example 17. The system of any of the preceding examples, wherein the simulating the one or more insertion configurations includes registering an axis of the 3D cochlea model with an axis of the one or more 3D electrode lead models.
Example 18. The system of any of the preceding examples, wherein the performing the operation includes determining an insertion depth of the one or more electrode leads.
Example 19. The system of any of the preceding examples, wherein the determining the insertion depth includes determining a first insertion depth of the one or more electrode leads at which the one or more electrode leads are released from a stylet.
Example 20. The system of any of the preceding examples, wherein the first insertion depth corresponds to a first marker on the one or more electrode leads.
Example 21. The system of any of the preceding examples, wherein the determining the insertion depth includes determining a second insertion depth of the one or more electrode leads at which the one or more electrode leads are fully insert within the cochlea.
Example 22. The system of any of the preceding examples, wherein the determining the insertion depth is based on a position of an axis of the one or more electrode leads relative to an axis of the cochlea.
Example 23. The system of any of the preceding examples, wherein the second insertion depth corresponds to a second marker on the one or more electrode leads relative to a round window of the cochlea.
Example 24. The system of any of the preceding examples, wherein the one or more electrode leads are one or more preformed electrode leads.
Example 25. A computer program product embodied in a non-transitory computer readable storage medium and comprising computer instructions for performing a process comprising: accessing a three-dimensional (3D) cochlea model representative of a cochlea of a patient within which an electrode lead included in a cochlear implant system is to be inserted during an electrode lead insertion procedure; accessing one or more 3D electrode lead models representative of one or more electrode leads configured to be inserted within the cochlea, the one or more 3D electrode lead models including one or more properties of the one or more electrode leads; simulating, based on the 3D cochlea model and the one or more 3D electrode lead models, one or more insertion configurations associated with insertion of the one or more electrode leads into the cochlea; and performing, based on the simulating, an operation associated with the electrode lead insertion procedure.
Example 26. A method comprising: accessing, by an electrode lead simulation system, a three-dimensional (3D) cochlea model representative of a cochlea of a patient within which an electrode lead included in a cochlear implant system is to be inserted during an electrode lead insertion procedure; accessing, by the electrode lead simulation system, one or more 3D electrode lead models representative of one or more electrode leads configured to be inserted within the cochlea, the one or more 3D electrode lead models including one or more properties of the one or more electrode leads; simulating, by the electrode lead simulation system and based on the 3D cochlea model and the one or more 3D electrode lead models, one or more insertion configurations associated with insertion of the one or more electrode leads into the cochlea; and performing, by the electrode lead simulation system and based on the simulating, an operation associated with the electrode lead insertion procedure.
Example 27. A system comprising: a memory storing instructions; and a processor configured to execute the instructions to perform a process comprising: accessing a three-dimensional (3D) cochlea model representative of a cochlea of a patient within which an electrode lead included in a cochlear implant system is to be inserted during an electrode lead insertion procedure; determining one or more properties of a plurality of types of electrode leads configured to be inserted within the cochlea; selecting, based on the 3D cochlea model and the one or more properties, a type of electrode lead included in the plurality of types of electrode leads to be inserted during the electrode lead insertion procedure; and providing electrode lead insertion information indicative of the type of electrode lead to be inserted during the electrode lead insertion procedure.
Example 28. The system of any of the preceding examples, wherein the one or more properties of the plurality of types of electrode leads includes one or more of a stiffness associated with one or more electrode leads, a material associated with one or more electrode leads, a size associated with one or more electrode leads, an elastic modulus associated with one or more electrode leads, a shear stress associated with one or more electrode leads, or a friction associated with one or more electrode leads.
Example 29. The system of any of the preceding examples, wherein the selecting the type of electrode lead is further based on one or more insertion factors including one or more of an insertion angle of the electrode lead, an anatomy of the cochlea, or a proximity of a facial nerve to a position of the electrode lead.
Example 30. The system of any of the preceding examples, wherein the selecting the type of electrode lead further includes weighting the one or more insertion factors.
Example 31. The system of any of the preceding examples, wherein the selecting the type of electrode lead is further based on a pre-operative audiogram of the patient.
Example 32. The system of any of the preceding examples, wherein the electrode lead insertion information includes one or more of the type of the electrode lead to be inserted, a size of the electrode lead to be inserted, a rotation of the electrode lead to be inserted, an insertion depth of the electrode lead to be inserted, an insertion angle of the electrode lead to be inserted, or an insertion speed of the electrode lead to be inserted.
Example 33. The system of any of the preceding examples, wherein the selecting the type of electrode lead is further based on simulating one or more insertion configurations associated with insertion of one or more electrode leads included in the plurality of types of electrode leads into the cochlea.
Example 34. The system of any of the preceding examples, wherein the simulating is based on the 3D cochlea model and one or more 3D electrode lead models representative of one or more electrode leads included in the plurality of types of electrode leads configured to be inserted within the cochlea, the one or more 3D electrode lead models including the one or more properties of the plurality of types of electrode leads.
Example 35. The system of any of the preceding examples, wherein each insertion configuration of the one or more insertion configurations includes one or more insertion parameters.
Example 36. The system of any of the preceding examples, wherein the one or more insertion parameters include one or more of a type of one or more electrode leads, a rotation of one or more electrode leads, an insertion depth of one or more electrode leads, an insertion angle of one or more electrode leads, or an insertion speed of one or more electrode leads.
Example 37. The system of any of the preceding examples, wherein the simulating the one or more insertion configurations includes varying the one or more insertion parameters during the simulating.
Example 38. A computer program product embodied in a non-transitory computer readable storage medium and comprising computer instructions for performing a process comprising: accessing a three-dimensional (3D) cochlea model representative of a cochlea of a patient within which an electrode lead included in a cochlear implant system is to be inserted during an electrode lead insertion procedure; determining one or more properties of a plurality of types of electrode leads configured to be inserted within the cochlea; selecting, based on the 3D cochlea model and the one or more properties, a type of electrode lead included in the plurality of types of electrode leads to be inserted during the electrode lead insertion procedure; and providing electrode lead insertion information indicative of the type of electrode lead to be inserted during the electrode lead insertion procedure.
Example 39. A method comprising: accessing, by an electrode lead simulation system, a three-dimensional (3D) cochlea model representative of a cochlea of a patient within which an electrode lead included in a cochlear implant system is to be inserted during an electrode lead insertion procedure; determining, by the electrode lead simulation system, one or more properties of a plurality of types of electrode leads configured to be inserted within the cochlea; selecting, by the electrode lead simulation system and based on the 3D cochlea model and the one or more properties, a type of electrode lead included in the plurality of types of electrode leads to be inserted during the electrode lead insertion procedure; and providing, by the electrode lead simulation system, electrode lead insertion information indicative of the type of electrode lead to be inserted during the electrode lead insertion procedure.
Example 40. A system comprising: a memory storing instructions; and a processor configured to execute the instructions to perform a process comprising: accessing a three-dimensional (3D) cochlea model representative of a cochlea of a patient within which an electrode lead included in a cochlear implant system is to be inserted during an electrode lead insertion procedure; accessing an 3D electrode lead model representative of a preformed electrode lead configured to be inserted within the cochlea, the 3D electrode lead model including one or more properties of the preformed electrode lead; registering an axis of the 3D cochlea model with an axis of the 3D electrode lead model; determining, based on the registering, an insertion depth associated with insertion of the preformed electrode lead into the cochlea; and providing electrode lead insertion information indicative of the insertion depth for the electrode lead insertion procedure.
Example 41. The system of any of the preceding examples, wherein the one or more properties of the preformed electrode lead includes one or more of a stiffness associated with the preformed electrode lead, a material associated with the preformed electrode lead, a size associated with the preformed electrode lead, an elastic modulus associated with the preformed electrode lead, a shear stress associated with the preformed electrode lead, or a friction associated with the preformed electrode lead.
Example 42. The system of any of the preceding examples, wherein the determining the insertion depth includes determining a first insertion depth of the one or more electrode leads at which the one or more electrode leads are released from a stylet.
Example 43. The system of any of the preceding examples, wherein the insertion depth corresponds to a first marker on the one or more electrode leads.
Example 44. The system of any of the preceding examples, wherein the determining the insertion depth includes determining a second insertion depth of the one or more electrode leads at which the one or more electrode leads are fully insert within the cochlea.
Example 45. The system of any of the preceding examples, wherein the determining the insertion depth is based on a position of the axis of the one or more electrode leads relative to the axis of the cochlea.
Example 46. The system of any of the preceding examples, wherein the insertion depth corresponds to a second marker on the one or more electrode leads relative to a round window of the cochlea.
Example 47. The system of any of the preceding examples, wherein the determining the insertion depth is further based on simulating one or more insertion configurations associated with insertion of the preformed electrode lead into the cochlea using the 3D cochlea model and the 3D electrode lead model.
Example 48. The system of any of the preceding examples, wherein each insertion configuration of the one or more insertion configurations includes one or more insertion parameters.
Example 49. The system of any of the preceding examples, wherein the one or more insertion parameters include one or more of a type of the preformed electrode lead, a rotation of the preformed electrode lead, an insertion depth of the preformed electrode lead, an insertion angle of the preformed electrode lead, or an insertion speed of the preformed electrode lead.
Example 50. The system of any of the preceding examples, wherein the simulating the one or more insertion configurations includes varying the one or more insertion parameters during the simulating.
Example 51. The system of any of the preceding examples, wherein the electrode lead insertion information further includes one or more of a type of the preformed electrode lead to be inserted, a size of the preformed electrode lead to be inserted, a rotation of the preformed electrode lead to be inserted, an insertion angle of the preformed electrode lead to be inserted, or an insertion speed of the preformed electrode lead to be inserted.
Example 52. A computer program product embodied in a non-transitory computer readable storage medium and comprising computer instructions for performing a process comprising: accessing a three-dimensional (3D) cochlea model representative of a cochlea of a patient within which an electrode lead included in a cochlear implant system is to be inserted during an electrode lead insertion procedure; accessing an 3D electrode lead model representative of a preformed electrode lead configured to be inserted within the cochlea, the 3D electrode lead model including one or more properties of the preformed electrode lead; registering an axis of the 3D cochlea model with an axis of the 3D electrode lead model; determining, based on the registering, an insertion depth associated with insertion of the preformed electrode lead into the cochlea; and providing electrode lead insertion information indicative of the insertion depth for the electrode lead insertion procedure.
Example 53. A method comprising: accessing, by an electrode lead simulation system, a three-dimensional (3D) cochlea model representative of a cochlea of a patient within which an electrode lead included in a cochlear implant system is to be inserted during an electrode lead insertion procedure; accessing, by the electrode lead simulation system, an 3D electrode lead model representative of a preformed electrode lead configured to be inserted within the cochlea, the 3D electrode lead model including one or more properties of the preformed electrode lead; registering, by the electrode lead simulation system, an axis of the 3D cochlea model with an axis of the 3D electrode lead model; determining, by the electrode lead simulation system and based on the registering, an insertion depth associated with insertion of the preformed electrode lead into the cochlea; and providing, by the electrode lead simulation system, electrode lead insertion information indicative of the insertion depth for the electrode lead insertion procedure.

In the preceding description, various exemplary embodiments have been described with reference to the accompanying drawings. It will, however, be evident that various modifications and changes may be made thereto, and additional embodiments may be implemented, without departing from the scope of the invention as set forth in the claims that follow. For example, certain features of one embodiment described herein may be combined with or substituted for features of another embodiment described herein. The description and drawings are accordingly to be regarded in an illustrative rather than a restrictive sense.

## Claims

1. A system comprising:
a memory storing instructions; and
a processor configured to execute the instructions to perform a process comprising:
accessing a three-dimensional (3D) cochlea model representative of a cochlea of a patient within which an electrode lead included in a cochlear implant system is to be inserted during an electrode lead insertion procedure;
accessing one or more 3D electrode lead models representative of one or more electrode leads configured to be inserted within the cochlea, the one or more 3D electrode lead models including one or more properties of the one or more electrode leads;
simulating, based on the 3D cochlea model and the one or more 3D electrode lead models, one or more insertion configurations associated with insertion of the one or more electrode leads into the cochlea; and
performing, based on the simulating, an operation associated with the electrode lead insertion procedure.

2. The system of claim 1, wherein the 3D cochlea model is based on pre-operative imaging of the cochlea of the patient.

3. The system of claim 1, wherein the one or more properties of the one or more electrode leads includes one or more of a stiffness associated with the one or more electrode leads, a material associated with the one or more electrode leads, a size associated with the one or more electrode leads, one or more elastic moduli associated with the one or more electrode leads, a shear stress associated with the one or more electrode leads, or a friction associated with the one or more electrode leads.

4. The system of claim 1, wherein each insertion configuration of the one or more insertion configurations includes one or more insertion parameters.

5. The system of claim 4, wherein the one or more insertion parameters include one or more of a type of the one or more electrode leads, a rotation of the one or more electrode leads, an insertion depth of the one or more electrode leads, an insertion angle of the one or more electrode leads, or an insertion speed of the one or more electrode leads; or wherein the simulating the one or more insertion configurations includes varying the one or more insertion parameters during the simulating; or wherein the one or more insertion parameters are designated based on a user input.

6. The system of claim 1, wherein the simulating the one or more insertion configurations includes simulating trauma to the cochlea for each insertion configuration.

7. The system of claim 6, wherein the performing the operation includes prioritizing the one or more insertion configurations based on reducing trauma to the cochlea such that a first insertion configuration associated with less trauma to the cochlea than a second insertion configuration is prioritized above the second insertion configuration; or wherein the performing the operation includes selecting an insertion configuration of the one or more insertion configurations based on reducing trauma to the cochlea.

8. The system of claim 1, wherein the performing the operation includes providing insertion configuration information for the electrode lead insertion procedure.

9. The system of claim 8, wherein the insertion configuration information includes one or more of a type of the electrode lead to be inserted, a rotation of the electrode lead to be inserted, an insertion depth of the electrode lead to be inserted, an insertion angle of the electrode lead to be inserted, or an insertion speed of the electrode lead to be inserted.

10. The system of claim 1, wherein the performing the operation includes selecting, based on one or more insertion factors, a type of electrode lead to be inserted during the electrode lead insertion procedure.

11. The system of claim 10, wherein the one or more insertion factors includes one or more of an insertion angle of the electrode lead, an anatomy of the cochlea, or a proximity of a facial nerve to a position of the electrode lead; or wherein the selecting the type of electrode lead includes weighting the one or more insertion factors; or wherein the selecting the type of electrode lead is further based on a pre-operative audiogram of the patient.

12. The system of claim 1, wherein the simulating the one or more insertion configurations includes registering an axis of the 3D cochlea model with an axis of the one or more 3D electrode lead models.

13. The system of claim 1, wherein the performing the operation includes determining an insertion depth of the one or more electrode leads.

14. The system of claim 13, wherein the determining the insertion depth includes determining a first insertion depth of the one or more electrode leads at which the one or more electrode leads are released from a stylet, in particular with the first insertion depth corresponding to a first marker on the one or more electrode leads; or wherein the determining the insertion depth includes determining a second insertion depth of the one or more electrode leads at which the one or more electrode leads are fully insert within the cochlea, in particular with the determining the insertion depth being based on a position of an axis of the one or more electrode leads relative to an axis of the cochlea, or with the second insertion depth corresponding to a second marker on the one or more electrode leads relative to a round window of the cochlea; or wherein the one or more electrode leads are one or more preformed electrode leads.

15. A computer program product embodied in a non-transitory computer readable storage medium and comprising computer instructions for performing a process comprising:
accessing a three-dimensional (3D) cochlea model representative of a cochlea of a patient within which an electrode lead included in a cochlear implant system is to be inserted during an electrode lead insertion procedure;
accessing one or more 3D electrode lead models representative of one or more electrode leads configured to be inserted within the cochlea, the one or more 3D electrode lead models including one or more properties of the one or more electrode leads;
simulating, based on the 3D cochlea model and the one or more 3D electrode lead models, one or more insertion configurations associated with insertion of the one or more electrode leads into the cochlea; and
performing, based on the simulating, an operation associated with the electrode lead insertion procedure.
